# EUROPÄISCHE PATENTSCHRIFT

(11) **EP 1 456 366 B1**
(45) Veröffentlichungstag und Bekanntmachung des Hinweises auf die Patenterteilung: **18.01.2012**
(21) Anmeldenummer: 02791811.9
(22) Anmeldetag: 12.12.2002
(51) Int. Cl.: C12N 9/54, C12N 5/10, A61K 8/66

(54) **NEUE ALKALISCHE PROTEASE AUS BACILLUS GIBSONII (DSM 14393) UND WASCH- UND REINIGUNGSMITTEL ENTHALTEND DIESE NEUE ALKALISCHE PROTEASE**
NOVEL ALKALI PROTEASE FORMED BY BACILLUS GIBSONII (DSM 14393) AND WASHING AND CLEANING AGENTS CONTAINING SAID NOVEL ALKALI PROTEASE
NOUVELLE PROTEASE ALCALINE EXTRAITE DE BACILLUS GIBSONII (DSM 14393) ET AGENTS DE LAVAGE ET DE NETTOYAGE CONTENANT CETTE NOUVELLE PROTEASE ALCALINE

(30) Priorität: 20.12.2001 DE 10162728
(43) Veröffentlichungstag der Anmeldung: 15.09.2004
(73) Patentinhaber: Henkel AG & Co. KGaA, 40589 Düsseldorf (DE)
(72) Erfinder: WEBER, Angrit, 51467 Bergisch-Gladbach (DE); HELLEBRANDT, Angela, 50735 Köln (DE); WIELAND, Susanne, 40589 Düsseldorf (DE); MAURER, Karl-Heinz, 40699 Erkrath (DE); KOTTWITZ, Beatrix, 40699 Erkrath (DE)
(86) Internationale Anmeldenummer: PCT/EP2002/014099
(87) Internationale Veröffentlichungsnummer: WO 2003/054184

(56) Entgegenhaltungen:
- WO-A-01/68821
- DE-A- 4 411 223
- SIEZEN R J ET AL: "SUBTILASES: THE SUPERFAMILY OF SUBTILISIN-LIKE SERINE PROTEASES" PROTEIN SCIENCE, CAMBRIDGE UNIVERSITY PRESS, CAMBRIDGE, GB, Bd. 6, Nr. 3, März 1997 (1997-03), Seiten 501-523, XP000856203 ISSN: 0961-8368 in der Anmeldung erwähnt

### SEQUENZPROTOKOLL

<110> Henkel Kommanditgesellschaft auf Aktien
<120> Neue Alkalische protease aus Bacillus gibsonii (DSM 14393) und Wasch- und Reinigungsmittel enthaltend diese neue Alkalische Protease
<130> H5390PCT
<140>
   <141>
<150> DE 10162728.9 <151> 2001-12-20
<160> 2
<170> PatentIn Ver. 2.1
<210> 1
   <211> 1152
   <212> DNA
   <213> Bacillus gibsonii (DSM 14393)
<220>
   <221> CDS
   <222> (1)..(1152)
<220>
   <221> mat_peptide
   <222> (343)..(1152)
<400> 1
   atg aaa aga aga gta gga aag ctt gta gtg ggg ctt gtt tgt gta aca    48
   Met Lys Arg Arg Val Gly Lys Leu Val Val Gly Leu Val Cys Val Thr
                   -110                -105                -100
   gct cta gta aca gta aca gat tct gca tct gca gca gaa gaa aag gta    96
   Ala Leu Val Thr Val Thr Asp Ser Ala Ser Ala Ala Glu Glu Lys Val
               -95                 -90                 -85
   aaa tac tta ata ggg ttt gaa gaa gaa gca gaa ctt gaa gcc ttc act    144
   Lys Tyr Leu Ile Gly Phe Glu Glu Glu Ala Glu Leu Glu Ala Phe Thr
           -80                 -75                 -70
   gag gaa att gac caa gtt ggt gtg ttt tct gtt gaa gaa caa agt gta    192
   Glu Glu Ile Asp Gln Val Gly Val Phe Ser Val Glu Glu Gln Ser Val
       -65                 -60                 -55
   gct gag gat acg tta gat att gat gta gac att att gat gaa tat gat    240
   Ala Glu Asp Thr Leu Asp Ile Asp Val Asp Ile Ile Asp Glu Tyr Asp
   -50                 -45                 -40                 -35
   tat att gat gta tta gct gta gaa tta gat cct gag gat gta gat gcg    288
   Tyr Ile Asp Val Leu Ala Val Glu Leu Asp Pro Glu Asp Val Asp Ala
                   -30                 -25                 -20
   tta agc gaa gaa gca ggt atc tca ttt att gaa gaa gac att gaa ctg    336
   Leu Ser Glu Glu Ala Gly Ile Ser Phe Ile Glu Glu Asp Ile Glu Leu
               -15                     -10                     -5
   tct atc caa caa acg gtt cct tgg ggc att act cgt gta caa gct cca     384
   Ser Ile Gln Gln Thr Val Pro Trp Gly Ile Thr Arg Val Gln Ala Pro
        -1   1               5                  10
   gct gta cat aac cga gga gta aca ggg tct ggt gta aga gta gcg att     432
   Ala Val His Asn Arg Gly Val Thr Gly Ser Gly Val Arg Val Ala Ile
    15                  20                  25                  30
   cta gat tca gga atc tct aca cat agt gat tta acg att cgc ggt gga     480
   Leu Asp Ser Gly Ile Ser Thr His Ser Asp Leu Thr Ile Arg Gly Gly
                    35                  40                  45
   gct agc ttt gta ccg ggt gaa cca aca acg gct gat tta aat ggt cat     528
   Ala Ser Phe Val Pro Gly Glu Pro Thr Thr Ala Asp Leu Asn Gly His
               50                  55                  60
   ggg acg cac gtt gca gga aca gtg gca gct ctt aat aat tca atc ggt     576
   Gly Thr His Val Ala Gly Thr Val Ala Ala Leu Asn Asn Ser Ile Gly
           65                  70                  75
   gtg att ggt gtg gca cca agt gct gat cta tac gct gta aaa gta ctt     624
   Val Ile Gly Val Ala Pro Ser Ala Asp Leu Tyr Ala Val Lys Val Leu
        80                  85                  90
   gga gca aat ggt aga gga agc gtt agt gga att gct caa ggt cta gag     672
   Gly Ala Asn Gly Arg Gly Ser Val Ser Gly Ile Ala Gln Gly Leu Glu
    95                 100                 105                 110
   tgg gct gca gcg aat aac atg cat att gct aac atg agt ctc ggt agt     720
   Trp Ala Ala Ala Asn Asn Met His Ile Ala Asn Met Ser Leu Gly Ser
                   115                 120                 125
   gat gca cct agt att aca ctt gag cgt gca gtc aac tac gcg aca agc     768
   Asp Ala Pro Ser Ile Thr Leu Glu Arg Ala Val Asn Tyr Ala Thr Ser
               130                 135                 140
   caa ggt gta cta gtt att gca gcg act ggt aac aac ggt tct ggt tca     816
   Gln Gly Val Leu Val Ile Ala Ala Thr Gly Asn Asn Gly Ser Gly Ser
           145                 150                 155
   gtt ggc tat cct gct cgt tat gca aac gca atg gct gta gga gcg act     864
   Val Gly Tyr Pro Ala Arg Tyr Ala Asn Ala Met Ala Val Gly Ala Thr
       160                 165                 170
   gac caa aac aac aga cgt gca aac ttt tct cag tat ggt aca gga att     912
   Asp Gln Asn Asn Arg Arg Ala Asn Phe Ser Gln Tyr Gly Thr Gly Ile
   175                 180                 185                 190
   gac atc gta gca cca ggg gtt aat gta caa agt acg tat cct gga aac     960
   Asp Ile Val Ala Pro Gly Val Asn Val Gln Ser Thr Tyr Pro Gly Asn
                   195                 200                 205
   cgc tat gca agt tta aat ggt aca tca atg gct act cca cac gta gct    1008
   Arg Tyr Ala Ser Leu Asn Gly Thr Ser Met Ala Thr Pro His Val Ala
               210                 215                 220
   ggt gcc gct gca ctt gta aag caa cgc tat cca tct tgg aat gca act    1056
   Gly Ala Ala Ala Leu Val Lys Gln Arg Tyr Pro Ser Trp Asn Ala Thr
           225                 230                 235
   caa att cgc aat cat ctg aaa aat aca gcg aca aat cta gga aac tct    1104
   Gln Ile Arg Asn His Leu Lys Asn Thr Ala Thr Asn Leu Gly Asn Ser
       240                 245                 250
   tcg caa ttt ggt agt ggc cta gtg aac gca gaa gca gca aca cgt taa    1152
   Ser Gln Phe Gly Ser Gly Leu Val Asn Ala Glu Ala Ala Thr Arg
   255                 260                 265                 270
<210> 2
   <211> 383
   <212> PRT
   <213> Bacillus gibsonii (DSM 14393)
<400> 2
   Met Lys Arg Arg Val Gly Lys Leu Val Val Gly Leu Val Cys Val Thr
   1               5                   10                  15
   Ala Leu Val Thr Val Thr Asp Ser Ala Ser Ala Ala Glu Glu Lys Val
               20                  25                  30
   Lys Tyr Leu Ile Gly Phe Glu Glu Glu Ala Glu Leu Glu Ala Phe Thr
           35                  40                  45
   Glu Glu Ile Asp Gln Val Gly Val Phe Ser Val Glu Glu Gln Ser Val
       50                  55                  60
   Ala Glu Asp Thr Leu Asp Ile Asp Val Asp Ile Ile Asp Glu Tyr Asp
   65                  70                  75                  80
   Tyr Ile Asp Val Leu Ala Val Glu Leu Asp Pro Glu Asp Val Asp Ala
                   85                  90                  95
   Leu Ser Glu Glu Ala Gly Ile Ser Phe Ile Glu Glu Asp Ile Glu Leu
               100                 105                 110
   Ser Ile Gln Gln Thr Val Pro Trp Gly Ile Thr Arg Val Gln Ala Pro
           115                 120                 125
   Ala Val His Asn Arg Gly Val Thr Gly Ser Gly Val Arg Val Ala Ile
       130                 135                 140
   Leu Asp Ser Gly Ile Ser Thr His Ser Asp Leu Thr Ile Arg Gly Gly
   145                 150                 155                 160
   Ala Ser Phe Val Pro Gly Glu Pro Thr Thr Ala Asp Leu Asn Gly His
                   165                 170                 175
   Gly Thr His Val Ala Gly Thr Val Ala Ala Leu Asn Asn Ser Ile Gly
               180                 185                 190
   Val Ile Gly Val Ala Pro Ser Ala Asp Leu Tyr Ala Val Lys Val Leu
           195                 200                 205
   Gly Ala Asn Gly Arg Gly Ser Val Ser Gly Ile Ala Gln Gly Leu Glu
       210                 215                 220
   Trp Ala Ala Ala Asn Asn Met His Ile Ala Asn Met Ser Leu Gly Ser
   225                 230                 235                 240
   Asp Ala Pro Ser Ile Thr Leu Glu Arg Ala Val Asn Tyr Ala Thr Ser
                   245                 250                 255
   Gln Gly Val Leu Val Ile Ala Ala Thr Gly Asn Asn Gly Ser Gly Ser
               260                 265                 270
   Val Gly Tyr Pro Ala Arg Tyr Ala Asn Ala Met Ala Val Gly Ala Thr
           275                 280                 285
   Asp Gln Asn Asn Arg Arg Ala Asn Phe Ser Gln Tyr Gly Thr Gly Ile
       290                 295                 300
   Asp Ile Val Ala Pro Gly Val Asn Val Gln Ser Thr Tyr Pro Gly Asn
   305                 310                 315                 320
   Arg Tyr Ala Ser Leu Asn Gly Thr Ser Met Ala Thr Pro His Val Ala
                   325                 330                 335
   Gly Ala Ala Ala Leu Val Lys Gln Arg Tyr Pro Ser Trp Asn Ala Thr
               340                 345                 350
   Gln Ile Arg Asn His Leu Lys Asn Thr Ala Thr Asn Leu Gly Asn Ser
           355                 360                 365
   Ser Gln Phe Gly Ser Gly Leu Val Asn Ala Glu Ala Ala Thr Arg
       370                 375                 380

## Beschreibung

Die vorliegende Erfindung betrifft eine neue Alkalische Protease vom Subtilisin-Typ aus *Bacillus gibsonii* (DSM 14393) sowie hinreichend verwandte Proteine und deren Derivate. Sie betrifft außerdem Wasch- und Reinigungsmittel mit dieser neuen Alkalischen Protease vom Subtilisin-Typ, hinreichend verwandten Proteinen und deren Derivaten, entsprechende Wasch- und Reinigungsverfahren und deren Verwendung in Wasch- und Reinigungsmitteln sowie weitere technische Einsatzmöglichkeiten.

Proteasen vom Subtilisin-Typ (Subtilasen, Subtilopeptidasen, EC 3.4.21.62), insbesondere Subtilisine werden aufgrund der katalytisch wirksamen Aminosäuren den Serin-Proteasen zugerechnet. Sie werden natürlicherweise von Mikroorganismen, insbesondere von *Bacillus*-Spezies gebildet und sekretiert. Sie wirken als unspezifische Endopeptidasen, das heißt sie hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegegeben von R. Bott und C. Betzel, New York, 1996. Subtilisine eignen sich für eine Vielzahl von technischen Verwendungsmöglichkeiten, als Bestandteile von Kosmetika und insbesondere als aktive Inhaltsstoffe von Wasch- oder Reinigungsmitteln.

Enzyme sind etablierte aktive Inhaltsstoffe von Wasch- und Reinigungsmitteln. Proteasen bewirken dabei den Abbau proteinhaltiger Anschmutzungen auf dem Reinigungsgut, wie beispielsweise Textilien oder harten Oberflächen. Günstigenfalls ergeben sich Synergieeffekte zwischen den Enzymen und den übrigen Bestandteilen der betreffenden Mittel. Dies ist beispielsweise in US 6008178 dargestellt. Unter den Waschund Reinigungsmittelproteasen nehmen Subtilisine aufgrund ihrer günstigen enzymatischen Eigenschaften wie Stabilität oder pH-Optimum eine herausragende Stellung ein.

Aus der internationalen Offenlegungsschrift WO 0168821 geht beispielsweise eine in den E. coli Stamm MT173 (DSM 13306) eingebrachte alkalische Protease hervor und deren Einsatzmöglichkeit in Wasch- du Reinigungsmitteln hervor.

In der deutschen Offenlegungsschrift DE 4411223 ist die Verwendung von Proteasen aus der Subtilisin/Elastase-Familie in Wasch- und Reinigungsverfahren offenbart.

Die Entwicklung von Waschmittelproteasen beruht auf natürlicherweise, vorzugsweise mikrobiell gebildeten Enzymen. Solche werden über an sich bekannte Mutagenese-Verfahren, beispielsweise Punktmutagenese, Deletion, Insertion oder Fusion mit anderen Proteinen oder Proteinteilen oder über sonstige Modifikationen für den Einsatz in Wasch- und Reinigungsmitteln optimiert.

Die diversen technischen Einsatzgebiete erfordern Proteasen mit unterschiedlichen Eigenschaften, was beispielsweise die Reaktionsbedingungen, die Stabilität oder die Substratspezifität angeht. Umgekehrt hängen die technischen Einsatzmöglichkeiten der Proteasen, beispielsweise im Kontext einer Wasch- oder Reinigungsmittelrezeptur, von weiteren Faktoren wie Stabilität des Enzyms gegenüber hohen Temperaturen, gegenüber oxidierenden Agentien, dessen Denaturierung durch Tenside, von Faltungseffekten oder von erwünschten Synergien mit anderen Inhaltsstoffen ab.

Somit besteht nach wie vor ein hoher Bedarf an technisch einsetzbaren Proteasen, die aufgrund der Vielzahl ihrer Einsatzgebiete in ihrer Gesamtheit ein breites Spektrum an Eigenschaften bis hin zu sehr subtilen Leistungsunterschieden abdecken.

Die Basis hierfür wird durch neue Proteasen erweitert, welche ihrerseits hinsichtlich spezieller Einsatzgebiete gezielt weiterentwickelt werden können.

Der vorliegenden Erfindung lag also die Aufgabe zugrunde, eine weitere, noch nicht bekannte Protease aufzufinden. Das Wildtyp-Enzym sollte sich vorzugsweise dadurch auszeichnen, daß es bei Verwendung in einem entprechenden Mittel den für diesen Zweck etablierten Enzymen zumindest nahekommt. Hierbei war insbesondere der Beitrag zur Leistung eines Wasch- oder Reinigungsmittels von Interesse.

Weitere Teilaufgaben bestanden darin, Nukleinsäuren zur Verfügung zu stellen, die für derartige Proteasen codieren, und Vektoren, Wirtszelle und Herstellverfahren zur Verfügung zu stellen, die zur Gewinnung derartiger Proteasen genutzt werden können. Ferner sollten entsprechende Mittel, insbesondere Wasch- und Reinigungsmittel, entsprechende Wasch- und Reinigungsverfahren sowie entsprechende Verwendungsmöglichkeiten für derartige Proteasen zur Verfügung gestellt werden. Schließlich sollten technische Einsatzmöglichkeiten für die gefundenen Proteasen definiert werden.

Die Lösung der Aufgabe besteht in Alkalischen Proteasen vom Subtilisin-Typ wie in den Ansprüchen definiert

Weitere Lösungen der Aufgabe, beziehungsweise Lösungen der Teilaufgaben und somit jeweils eigene Erfindungsgegenstände bestehen in Nukleinsäuren, die für erfindungsgemäße Proteasen codieren, in entsprechenden Vektoren, Zellen, beziehungsweise Wirtszellen und Herstellverfahren. Ferner werden entsprechende Mittel, insbesondere Wasch- und Reinigungsmittel, entsprechende Wasch- und Reinigungsverfahren sowie entsprechende Verwendungsmöglichkeiten für derartige Proteasen zur Verfügung gestellt. Schließlich werden technische Einsatzmöglichkeiten für die gefundenen Proteasen definiert.

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürliche vorkommenden L-Aminosäuren mit den international gebräuchlichen 1- und 3-Buchstaben-Codes bezeichnet. Die Kombination einer dieser Bezeichnungen mit einer Nummer bezeichnet für das jeweilige Protein, welchen Aminosäure-Rest es in der jeweiligen Position trägt. Für Punktmutationen sind analoge Bezeichnungen etabliert. Positionsangaben beziehen sich, soweit nicht anders angegeben, auf die jeweils maturen Formen der betreffenden Proteine, also ohne die Signalpeptide (siehe unten).

Unter einem **Enzym** ist im Sinne der vorliegenden Anmeldung ein Protein zu verstehen, das eine bestimmte biochemische Funktion ausübt. Beispielsweise unter **proteolytischen Enzymen** oder Enzymen mit proteolytischer Funktion sind im allgemeinen solche zu verstehen, die die Säureamidbindungen von Proteinen hydrolysieren, insbesondere solche, die im Inneren der Proteine liegen, und deshalb auch als endo-Peptidasen bezeichnet werden können. Subtitisin-Proteasen sind solche endo-Peptidasen, die natürlicherweise von gram-positiven Bakterien gebildet und zumeist sekretiert werden, oder von diesen, beispielsweise über molekularbiologische Methoden abgeleitet sind und sich über Teilbereiche, wie strukturbildende oder funktionstragende Regionen mit den natürlichen Subtilisin-Proteasen homologisieren lassen. Sie werden den Subtilasen zugeordnet. Diese werden beispielsweise in dem Artikel "Subtilases: Subtilisin-like Proteases" von R. Siezen, Seite 75-95 in "Subtilisin enzymes", herausgegegeben von R. Bott und C. Betzel, New York, 1996, dargestellt. Zahlreiche Proteine werden als sogenannte **Präproteine**, also zusammen mit einem **Signalpeptid** gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedigungen durch eine Signalpeptidase vom übrigen Protein abgespalten, so daß dieses seine eigentliche katalytische Aktivität ohne die zunächst vorhandenen N-terminalen Aminosäuren ausübt.

Für technische Anwendungen sind aufgrund ihrer enzymatischen Aktivität die **maturen Peptide**, das heißt die nach ihrer Herstellung prozessierten Enzyme gegenüber den Präproteinen bevorzugt.

**Pro-Proteine** sind inaktive Vorstufen von Proteinen. Deren Vorläufer mit Signalsequenz werden als **Prä-Pro-Proteine** bezeichnet.

Unter **Nukleinsäuren** sind im Sinne der vorliegenden Anmeldung die natürlicherweise aus Nukleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen. Aus ihnen können beispielsweise über reverse Transkription wiederum (c-)DNA-Moleküle abgeleitet werden.

Die einem Protein entsprechende Informationseinheit einer Nukleinsäure wird auch im Sinne der vorliegenden Anmeldung als **Gen** bezeichnet. Bei DNA sind die Sequenzen beider komplementärer Stränge in jeweils allen drei möglichen Leserastem zu berücksichtigen. Ferner ist zu berücksichtigen, daß verschiedene Codon-Tripiets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdern weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen und angegebene Nukleotidsequenzen jeweils nur als eine beispielhafte Codierung für eine bestimmte Aminosäurefolge angesehen werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen vollständige Gene herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt. Dies ist insbesondere dann möglich, wenn auf einen bei einer Stammsammlung hinterlegten Stamm zurückgegriffen werden kann. Beispielsweise mit PCR-Primem, die anhand einer bekannten Sequenz synthetisierbar sind, und/oder über isolierte mRNA-Moleküle können aus solchen Stämmen die betreffenden Gene synthetisiert, kloniert und gewünschtenfalls weiter bearbeitet, beispielsweise mutagenisiert werden.

Änderungen der Nukleotidsequenz, wie sie beispielsweise durch an sich bekannte molekularbiologische Methoden herbeigeführt werden können, werden als **Mutationen** bezeichnet. Je nach Art der Änderung kennt man beispielsweise **Deletions-**, **Insertions-** oder **Substitutionsmutationen** oder solche, bei denen verschiedene Gene oder Teile von Genen miteinander **fusioniert (shuffling)** werden; dies sind Genmutationen. Die zugehörigen Organismen werden als **Mutanten** bezeichnet. Die von mutierten Nukleinsäuren abgeleiteten Proteine werden als **Varianten** bezeichnet. So führen beispielsweise Deletions-, Insertions-, Substitutionsmutationen oder Fusionen zu deletions-, insertions-, substitutionsmutierten oder Fusionsgenen und auf Proteinebene zu entsprechenden Deletions-, Insertions- oder Substitutionsvarianten, beziehungsweise Fusionsproteinen.

Unter **Vektoren** werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten **Klonierungsvektoren,** und andererseits denen, die die Funktion erfülllen, das interessierende Gen in der Wirtszelle zu realisieren, das heißt, die Expression des betreffenden Proteins zu ermöglichen. Diese Vektoren werden als **Expressionsvektoren** bezeichnet.

Sowohl Bakterienzellen als auch eukaryontische Zellen, die die genannten Vektoren enthalten, werden ungeachtet ihrer Unterschiede allgemein als **Zellen** bezeichnet. Solche Zellen, die einen Vektor, insbesondere einen Expressionsvektor enthalten und somit zur Expression eines Transgens angeregt werden können, werden als **Wirtszellen** bezeichnet, denn sie beherbergen das betreffende genetische System.

**Homologisierung** ist der Vergleich einer Nukleinsäure- oder Aminosäuresequenz mit der von bekannten Genen oder Proteinen. Sie wird beispielsweise über ein Alignment vorgenommen. Das Maß für die Homologie ist ein Prozentsatz an **Identität,** wie er beispielsweise nach der von D. J. Lipman und W. R. Pearson in Science 227 (1985), S. 1435-1441 angegebenen Methode bestimmt werden kann. Diese Angabe kann sich auf das gesamte Protein oder auf den jeweils zuzuordnenden Bereich beziehen. Ein weiter gefaßter Homologie-Begriff, die **Ähnlichkeit,** bezieht auch konservierte Variationen, also Aminosäuren mit ähnlicher chemischer Aktivität in die Betrachtung mit ein, da diese innerhalb des Proteins meist ähnliche chemische Aktivitäten ausüben. Bei Nukleinsäuren kennt man nur den Prozentsatz an Identität.

Durch Homologisierung lassen sich aus der Aminosäure- oder Nukleotid-Sequenz die Funktionen einzelner Sequenzbereiche sowie die enzymatische Aktivität des betrachteten gesamten Enzyms folgern. Homologe Bereiche von verschiedenen Proteinen sind solche mit vergleichbaren Funktionen, die sich durch Identität oder konservierte Austausche in der primären Aminosäuresequenz erkennen lassen. Sie umfassen einzelne Aminosäuren, kleinste Bereiche, sogenannte Boxen, die wenige Aminosäuren lang sind, bis hin zu langen Bereichen in der primären Aminosäuresequenz. Unter den Funktionen der homologen Bereiche sind somit auch kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes. Andere Bereiche des Proteins, die nicht an der eigentlichen enzymatischen Reaktion beteiligt sind, können sie qualitativ oder quantitativ modifizieren. Dies betrifft beispielsweise die Enzymstabilität, die Aktivität, die Reaktionsbedingungen oder die Substratspezifität.

Unter dem Begriff eines **proteolytischen Enzyms** oder dem einer **Protease** sind deshalb über die Funktionen der wenigen Aminosäurereste des katalytisch aktiven Zentrums hinaus alle Funktionen zu verstehen, wie sie sich durch das Einwirken des gesamten übrigen Proteins oder eines Teils oder mehrerer Teile des übrigen Proteins auf die eigentlich katalytisch aktiven Bereiche ergeben. Auch allein solche modifizierenden Funktionen oder Teilaktivitäten werden, sofern sie eine Proteolyse-Reaktion unterstützen, im Sinne der Erfindung als proteolytische Aktivität angesehen. Zu solchen Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische Aktivität. Dabei kann es sich beispielsweise auch um die Ausbildung eines Strukturelements handeln, das fern vom aktiven Zentrum liegt. Die zweite Voraussetzung dafür, daß es sich erfindungsgemäß um ein proteolytisches Protein handelt, ist allerdings, daß sich durch das chemische Verhalten der eigentlich aktiven Reste allein oder zusätzlich durch das Einwirken der modifizierenden Teile eine Hydrolyse von Peptid-Bindungen ergibt. Es ist darüberhinaus möglich, daß auch die Aktivitäten anderer Proteasen durch einen oder mehrere Teile, beispielsweise des erfindungsgemäßen Proteins qualitativ oder quantitativ modifiziert werden. Diese Beeinflussung anderer Faktoren wird ebenfalls als proteolytische Aktivität angesehen. Proteolytisch aktive Enzyme sind auch solche Proteasen, deren Aktivität zu einem gegebenen Zeitpunkt, etwa durch einen Inhibitor blockiert ist. Entscheidend ist ihre prinzipielle Eignung zur entsprechenden Proteolyse-Reaktion.

Unter **Fragmenten** werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Unter **chimären** oder **hybriden** Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder Fusionsmutagenese genannt. Der Sinn einer solchen Fusion besteht beispielsweise darin, mithilfe des heranfusionierten erfindungsgemäßen Proteinteils eine enzymatische Funktion herbeizuführen oder zu modifizieren.

Unter durch **Insertionsmutation** erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist:der Anteil an Fremdprotein geringer als in chimären Proteinen.

**Inversionsmutagenese**, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter **Derivaten** werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können beispielsweise molekularbiologische Methoden, etwa die Cotransformation mit Genen, die für die betreffende Modifikation sorgen, eingesetzt werden. Derivatisierungen können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifikationen beeinflussen beispielsweise die Substratspezifität oder die Bindungsstärke an das Substrat oder führen eine vorübergehende Blockierung der enzymatischen Aktivität herbei, wenn es sich bei der angekoppelten Substanz um einen Inhibitor handelt. Dies ist beispielsweise für den Zeitraum der Lagerung sinnvoll. Ebenso ist unter Derivatisierung die kovalente Bindung an einen makromolekularen Träger zu verstehen.

Im Sinne der vorliegenden Erfindung werden alle Enzyme, Proteine, Fragmente, Fusionsproteine und Derivate, sofern sie nicht explizit als solche angesprochen zu werden brauchen, unter dem **Oberbegriff Proteine** zusammengefaßt.

Unter der **Leistung eines Enzyms** wird dessen Wirksamkeit im jeweils betrachteten technischen Bereich, vorzugsweise im Rahmen eines entsprechend ausgerichteten Mittels verstanden. Diese basiert auf der eigentlichen enzymatischen Aktivität, hängt darüberhinaus aber von weiteren, für den jeweiligen Prozeß relevanten Faktoren ab. Dazu gehören beispielsweise Stabilität, Substratbindung, Wechselwirkung mit dem das Substrat tragenden Material oder Wechselwirkungen mit anderen Inhaltsstoffen, insbesondere Synergien.

Unter der **Waschleistung** oder der **Reinigungsleistung eines Wasch-**, **beziehungsweise Reinigungsmittels** ist im Sinne der vorliegenden Anmeldung der Effekt zu verstehen, den das betrachtete Mittel auf die verschmutzten Artikel, beispielsweise Textilien oder Gegenstände mit harten Oberflächen ausübt. Einzelne Komponenten solcher Mittel, beispielsweise einzelne Enzyme, werden hinsichtlich ihres **Beitrags zur Wasch- oder Reinigungsleistung** des gesamten Wasch-, beziehungsweise Reinigungsmittels beurteilt. Denn aus den enzymatischen Eigenschaften eines Enzyms kann nicht ohne weiteres auf seinen Beitrag zur Waschleistung eines Mittels geschlossen werden. Hier spielen als weitere Faktoren beispielsweise Stabilität, Substratbindung, Bindung an das Reinigungsgut oder Wechselwirkungen mit anderen Inhaltsstoffen der Wasch- oder Reinigungsmittel, insbesondere Synergien bei der Entfernung der Verschmutzungen eine Rolle.

Die der vorliegenden Erfindung zugrunde liegende, natürlicherweise gebildete Alkalische Protease vom Subtilisin-Typ ist, wie anhand der Beispiele nachvollzogen werden kann, aus dem Kulturüberstand des Stammes ***Bacillus gibsonii* (DSM 14393)** erhältlich.

Dieser Stamm ist gemäß dem Budapester Vertrag über die internationale Anerkennung der **Hinterlegung von Mikroorganismen** vom 28. April 1977 am 1.3.2001 bei der Deutschen Sammlung von Mikroorganismen und Zellkulturen GmbH, Mascheroder Weg 1b, 38124 Braunschweig (http://www.dsmz.de), hinterlegt worden. Er trägt dort die Bezeichnung ID 01-194 und die Eingangsnummer DSM 14393. Die maßgeblichen Angaben über die Merkmale dieses biologischen Materials, wie sie von der DSMZ am 19.4.2001 bei der Hinterlegung bestimmt worden sind, werden in Tabelle 1 (Beispiel 1) zusammengestellt.

Die vorliegende Patentanmeldung hat die Strategie verfolgt, aus einem natürlichen Habitat einen Protease-bildenden Mikroorganismus und somit ein natürlicherweise gebildetes Enzym aufzufinden, das die gestellten Anforderungen möglichst vollständig erfüllt.

Solch ein Enzym konnte, wie in den Beispielen der vorliegenden Anmeldung beschrieben ist, mit der Alkalischen Protease aus *Bacillus gibsonii* (DSM 14393) gefunden werden.

Wie über die von der Deutschen Sammlung von Mikroorganismen und Zellkulturen GmbH angestellte, und in Tabelle 1 von Beispiel 1 angegebene biochemische Charakterisierung hinaus festgestellt werden kann, sekretiert dieser Stamm eine proteolytische Aktivität. Diese ist nach den Ausführungsbeispielen der vorliegenden Anmeldung untersucht worden und läßt sich folgendermaßen beschreiben: Es handelt sich gemäß SDS-Polyacrylamidgelelektrophorese um ein 26 kD-Protein, mit einem gemäß isoelektrischer Fokussierung bestimmten isoelektrischen Punkt von 11. Die spezifische Aktivität für das Substrat AAPF beträgt 16 U/mg. Das bei 50°C bestimmte pH-Optimum liegt bei pH 10.

Die Nukleotidsequenz der erfindungsgemäßen neuen Alkalischen Protease aus *Bacillus gibsonii* (DSM 14393) ist im Sequenzprotokoll der vorliegenden Anmeldung unter SEQ ID NO. 1 angegeben. Es umfaßt 1152 bp. Die hiervon abgeleitete Aminosäuresequenz wird in SEQ ID NO. 2 angegeben. Sie umfaßt 383 Aminosäuren, gefolgt von einem Stop-Codon. Hiervon sind die ersten 114 Aminosäuren wahrscheinlich nicht im maturen Protein enthalten, so daß sich für das mature Protein voraussichtlich eine Länge von 269 Aminosäuren ergibt.

Wie in Beispiel 2 beschrieben ist, wurden diese Sequenzen mit den aus allgemein zugänglichen Datenbanken Swiss-Prot (Geneva Bioinformatics (GeneBio) S.A., Genf, Schweiz; http:/lwww.genebio.com/sprot.html) und GenBank (National Center for Biotechnology Information NCBI, National Institutes of Health, Bethesda, MD, USA) erhältlichen Proteasesequenzen verglichen.

Hierüber wurden auf der Ebene der DNA für das gesamte Gen folgende drei Gene als nächstähnliche identifiziert: (1.) die Alkalische Elastase aus *Bacillus Ya-B* (ID ELYA_BACSP) mit 66% Identität, (2.) das Subtilisin Sendai aus *Bacillus Sendai* (ID Q45522) mit 65% Identität und (3.) das Subtilisin P92 aus *Bacillus alkalophilus* (ID ELYA_BACAO) mit 62% Identität.

Auf der Ebene der Aminosäuren für das gesamte Präproprotein wurden als nächstähnliche identifiziert: (1.) die Alkalische Elastase aus *Bacillus Ya-B* (ID ELYA_BACSP), gleichauf mit dem Subtilisin P92 aus *Bacillus alkalophilus* (ID ELYA_BACAO) mit 67% Identität, (2.) das Subtilisin Sendai aus *Bacillus Sendai* (ID Q45522) mit 65% Identität und (3.) das Subtilisin AprQ aus *Bacillus sp.* (ID Q45523) mit 49% Identität

Auf der Ebene der Aminosäuren für das mature Protein wurden als nächstähnliche identifiziert: (1.) die drei Subtilisine Subtilisin P92 aus *Bacillus alkalophilus* (ID ELYA_BACAO), Subtilisin 309 (Savinase^{®}) aus *Bacillus lentus* (ID SUBS_BACLE) und die *B. lentus*-Alkalische Protease aus *Bacillus lentus* DSM 5483 (ID SUBB_BACLE) mit jeweils 80% Identität, (2.) die beiden SubtilisineAlkalische Elastase aus *Bacillus Ya-B* (ID ELYA_BACSP) und Subtilisin Sendai aus *Bacillus Sendai* (ID Q45522) mit 78% Identität und (3.) das Subtilisin AprQ aus *Bacillus sp.* (ID Q45523) mit 61% Identität.

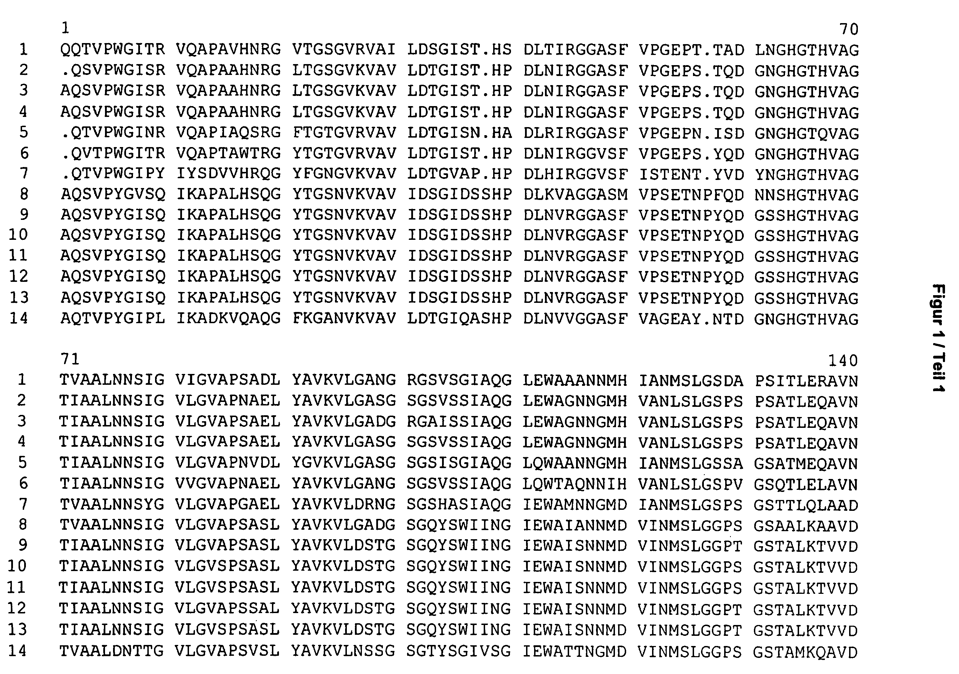

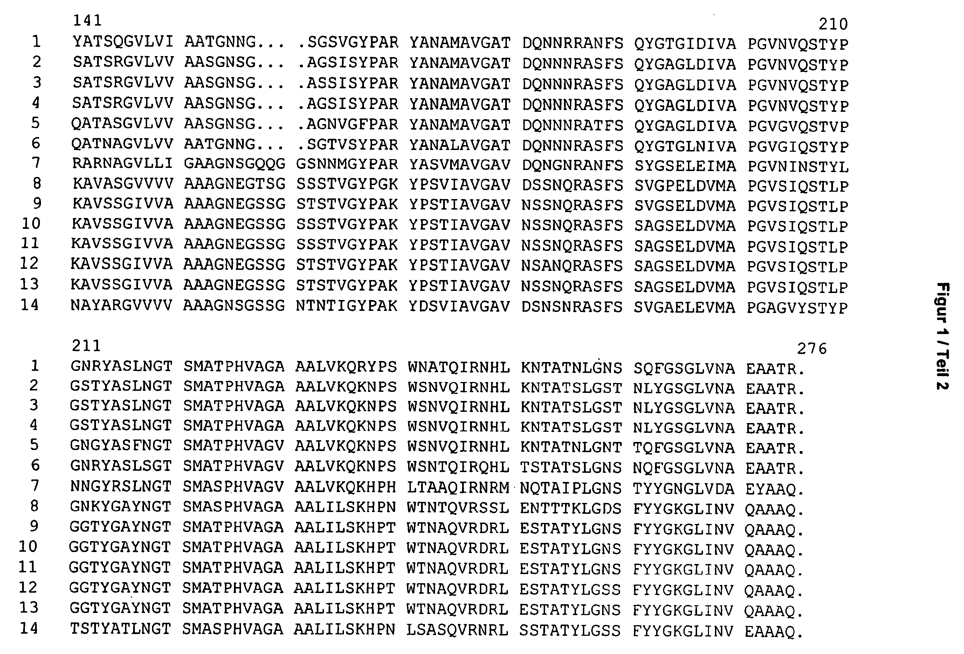

Weitere ähnliche Enzyme sind in Tabelle 2 in Beispiel 2 zusammengestellt und werden in dem Alignment der Figur 1 hinsichtlich der Aminosäuresequenzen der maturen Proteine mit der erfindungsgemäßen Alkalischen Protease aus *Bacillus gibsonii* (DSM 14393) verglichen.

Aufgrund der zu erkennenden Übereinstimmungen und der Verwandtschaft zu den anderen angegebenen Subtilisinen ist diese Alkalische Protease als ein Subtilisin anzusehen.

Ein Gegenstand der vorliegenden Erfindung ist somit jede Alkalische Protease vom Subtilisin-Typ mit einer Aminosäuresequenz, die zu der in SEQ ID NO. 2 angegebenen Aminosäuresequenz identisch ist.

Wie bereits erwährt, sind aufgrund eines Vergleichs der N-terminalen Sequenzen vermutlich die Aminosäuren 1 bis 114 als Leaderpeptid anzusehen und erstreckt sich das mature Protein voraussichtlich von den Positionen 115 bis 384 gemäß der SEQ ID NO. 2. Die Position 384 wird demnach von einem Stop-Codon eingenommen, entspricht also eigentlich keiner Aminosäure. Da aber auch die Information über das Ende eines codierenden Bereichs als wichtiger Bestandteil einer Aminosäuresequenz angesehen werden kann, wird diese Position erfindungsgemäß in den Bereich einbezogen, der dem maturen Protein entspricht.

Eine Ausführungsform dieses Erfindungsgegenstands ist somit jede Alkalische Protease vom Subtilisin-Typ mit einer Aminosäuresequenz, die zu der in SEQ **ID** NO.2 angegebenen Aminosäuresequenz in den Positionen 115 bis 384 identisch ist.

Sollte sich, beispielsweise durch eine N-terminale Sequenzierung des *in vivo* von *Bacillus gibsonii* (DSM 14393) freigesetzten proteolytischen Proteins herausstellen, daß die Spaltstelle nicht zwischen der 114. und der 115. Aminosäure gemäß SEQ ID NO. 2 sondern an einer anderen Stelle liegt, so beziehen sich diese Angaben auf das tatsächliche mature Protein.

Eine Ausführungsform dieses Erfindungsgegenstands ist jede Alkalische Protease vom Subtilisin-Typ, die sich von einer Nukleotidsequenz ableitet, die zu der in SEQ ID NO. 1 angegebenen Nukleotidsequenz identisch ist, insbesondere über den Teilbereich, der den Positionen 115 bis 384 gemäß der SEQ ID NO. 2 entspricht.

Auch hier, wie für alle nachfolgenden Ausführungsformen gilt wiederum, daß sich diese Angaben auf das tatsächliche mature Protein beziehen, falls sich herausstellen sollte, daß die Spaltstelle des Proteins an einer anderen Stelle als oben angegeben liegt.

Diese ist eine im Stand der Technik noch nicht bekannte Protease. Sie ist, wie in den Beispielen angegeben isolierbar, herstellbar und einsetzbar. Sie zeichnet sich, wie ebenfalls in den Beispielen dokumentiert, zusätzlich dadurch aus, daß sie bei Verwendung in einem entsprechenden Mittel den für diesen Zweck etablierten Enzymen in der Leistung nahekommt, beziehungsweise sie teilweise sogar übertrifft.

Für die Entwicklung von technischen, insbesondere in Waschmitteln einsetzbaren Proteasen kann sie als ein natürlicherweise, mikrobiell gebildetes Enzym als Ausgangspunkt dienen, um über an sich bekannte Mutagenese-Verfahren, beispielsweise Punktmutagenese, Fragmentierung, Deletion, Insertion oder Fusion mit anderen Proteinen oder Proteinteilen oder über sonstige Modifikationen für den gewünschten Einsatz optimiert zu werden. Derartige Optimierungen können beispielsweise Anpassungen an Temperatur-Einflüsse, pH-Wert-Schwankungen, Redox-Verhältnissen und/oder sonstigen Einflüsse, sein, die für die technischen Einsatzgebiete relevant sind. Gewünscht sind beispielsweise eine Verbesserung der Oxidationsbeständigkeit, der Stabilität gegenüber denaturierenden Agentien oder proteolytischem Abbau, gegenüber hohen Temperaturen, sauren oder stark alkalischen Bedingungen, eine Veränderung der Sensitivität gegenüber Calciumionen oder anderen Cofaktoren, eine Senkung der Immunogenität oder der allergenen Wirkung.

Hierfür können beispielsweise in Anwendung der Lehre von WO 00/36069 durch gezielte Punktmutationen die Oberflächenladungen oder die an der Katalyse oder Substratbindungen beteiligten Loops geändert werden. Letzteres ist beispielsweise in WO 95/30011, WO 99/27082, WO 00/37599, WO 00/37621 bis WO 00/37627 und WO 00/1683 bis WO 00/71691 offenbart. Weitere, insbesondere über gentechnische Methoden einzuführende Modifikationen sind beispielsweise in Anwendung der Lehren aus den Anmeldungen WO 92/21760 und WO 95123221 durchführbar. Ein Ausgangspunkt hierfür ist das in Figur 1 der vorliegenden Anmeldung dargestellte Alignment. Dieses ermöglicht, aus den bekannten Enzymen interessante, in den genannten Anmeldungen beschriebene Positionen für die Protease aus *Bacillus gibsonii*

(DSM 14393) abzuleiten und nach an sich bekannten Methoden entsprechend zu variieren.

Die Mutagenese-Verfahren beruhen auf der zugehörigen Nukleotidsequenz, die in SEQ ID NO. 1 angegeben ist, die weiter unten als eigener Erfindungsgegenstand darstellt wird Entsprechende molekularbiologische Methoden sind im Stand der Technik beschrieben, so beispielsweise in Handbüchem wie dem von Fritsch, Sambrook und Maniatis "Molecular cloning: a laboratory manual", Cold Spring Harbour Laboratory Press, New York, 1989.

Weitere Ausführungsformen der vorliegenden Erfindung sind alle von einer oben beschriebenen, erfindungsgemäßen Alkalischen Protease vom Subtilisin-Typ durch Fragmentierung oder Deletionsmutagenese abgeleiteten Proteine oder Fragmente mit zunehmend bevorzugt mindestens 260, bereits im Ausgangsmolekül zusammenhängenden, intern der Ausgangsaminosäuresequenz gelegenen Aminosäuren.

Aus dem Alignment der Figur 1 geht mit dem Bereich von Position 214 bis 236 (nach der Zählung des Alignments) ein Fragment von 23 Aminosäuren Länge hervor, das mit dem nächstähnlichen Enzym identisch ist. Erfindungsgemäße durch Fragmentierung oder Deletionsmutagenese abgeleitete Proteine oder Fragmente besitzen folglich größere, nicht mit bekannten Proteasen identische Bereiche.

Dabei handelt es bevorzugt um solche, die dem Bereich der Aminosäuren 115 bis 384 gemäß SEQ ID NO. 2, das heißt dem maturen Protein entsprechen.

Unter erfindungsgemäßen Fragmenten werden alle Proteine oder Peptide verstanden, die kleiner sind als jene Proteine, die SEQ ID NO. 2 entsprechen, mit ihnen aber in den entsprechenden Teilsequenzen übereinstimmen. Solche Fragmente können kostengünstiger herzustellen sein, bestimmte eventuell nachteilige Charakteristika des Ausgangsmoleküls nicht mehr besitzen, wie möglicherweise einen aktivitätssenkenden Regulationsmechanismus, oder ein günstigeres Aktivitätsprofil entfalten. Derartige Proteinfragmente können auch nichtbiosynthetisch, sondern beispielsweise chemisch hergestellt werden. Die chemische Synthese ist beispielsweise dann vorteilhaft, wenn im Anschluß an die Synthese chemische Modifikationen vorgenommen werden sollen.

Den Fragmenten sind ihrer prinzipiellen Gleichartigkeit wegen auch durch Deletionsmutation erhältliche Proteine zuzuordnen. Die Deletionsmutagenese ist zur Entfernung inhibierender Bereiche besonders sinnvoll. Im Ergebnis können mit den Deletionen sowohl eine Spezialisierung als auch eine Erweiterung des Anwendungsbereichs des Proteins einhergehen.

Als natürlicherweise gebildete Fragmente, beziehungsweise deletionsmutierte Proteine kann man auch die von Präproteinen durch Abspaltung der N-terminaten Aminosäuren erhältlichen Proteine und Signalpeptide ansehen. Solch ein Spaltungsmechanismus kann beispielsweise dazu verwendet werden, um mithilfe bestimmter Sequenzbereiche, die von Signalpeptidasen erkannt werden, in rekombinanten Proteinen spezifische Spaltstellen vorzugeben. Somit können *in vitro* Aktivierung und/oder Deaktivierung erfindungsgemäßer Proteine vorgenommen werden.

Eine bevorzugte Ausführungsform stellen all solche bislang ausgeführten Proteine dar, die dadurch gekennzeichnet sind, daß sie zusätzlich derivatisiert sind.

Unter Derivaten werden solche Proteine verstanden, die sich über eine zusätzliche Modifikation von den ausgeführten Proteinen ableiten. Derartige Modifikationen können beispielsweise die Stabilität, Substratspezifität oder die Bindungsstärke an das Substrat oder die enzymatische Aktivität beeinflussen. Sie können auch dazu dienen, um die Allergenizität und/oder lmmunogenizität des Proteins herabzusetzen und damit beispielsweise dessen Hautverträglichkeit zu erhöhen.

Solche Derivatisierungen können beispielsweise biologisch erfolgen, etwa im Zusammenhang mit der Proteinbiosynthese durch den produzierenden Wirtsorganismus. Hier sind Kopplungen niedrigmolekularer Verbindungen wie von Lipiden oder Oligosacchariden besonders hervorzuheben.

Derivatisierungen können aber auch chemisch durchgeführt werden, etwa durch die chemische Umwandlung einer Seitenkette oder durch kovalente Bindung einer anderen, beispielsweise makromolekularen Verbindung an das Protein. Eine chemische Modifikation wird beispielsweise in der Anmeldung DE 4013142 beschrieben. Beispielsweise die Kopplung von Aminen an Carboxylgruppen eines Enzyms zur Veränderung des isoelektrischen Punkts geht aus WO 95/26398 hervor. Es können beispielsweise Makromoleküle wie Proteine, etwa über bifunktionelle chemische Verbindungen an erfindungsgemäße Proteine gebunden werden. So ist es beispielsweise in Anwendung der Lehre von WO 99/57154 bis WO 99/57159, WO 00/18865 und WO 00/57155 möglich, ein erfindungsgemäßes Protein über einen Linker mit einer spezifischen Bindungsdomäne zu versehen. Solche Derivate eignen sich besonders für den Einsatz in in Wasch- oder Reinigungsmitteln. Analog WO 00/01831 können auch Protease-Inhibitoren über Linker, insbesondere Aminosäure-Linker an die erfindungsgemäßen Proteine gebunden werden. Kopplungen mit sonstigen makromolekularen Verbindungen, wie etwa Polyethylenglykol verbessern das Molekül hinsichtlich weiterer Eigenschaften wie Stabilität oder Hautverträglichkeit. Solch eine Modifikation wird beispielsweise in dem Patent US 5230891 für Proteasen für den Einsatz in Kosmetika beschrieben.

Unter Derivaten erfindungsgemäßer Proteine können im weitesten Sinne auch Präparationen dieser Enzyme verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit bestimmten anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine proteolytische Funktion entfaltet. Dies kann beispielsweise über entsprechende Begleitstoffe gesteuert werden. Insbesondere die gemeinsame Präparation von Proteasen mit Protease-Inhibitoren ist aus dem Stand der Technik bekannt (WO 00101826).

Eine bevorzugte Ausführungsform stellen all solche bislang ausgeführten Proteine, Proteinfragmente oder Fusionsproteine dar, die dadurch gekennzeichnet sind, daß sie zusätzlich stabilisiert sind.

Dadurch wird ihre Stabilität bei der Lagerung und/oder während ihres Einsatzes, beispielsweise beim Waschprozeß erhöht, so daß ihre Aktivität länger anhält und damit verstärkt wird. Die Stabilität erfindungsgemäßer Proteasen wird durch Kopplung an Polymere erhöht. Solch eine Methode wird beispielsweise in US 5230891 beschrieben. Sie erfordert, daß die Proteine vor ihrem Einsatz in entsprechenden Mitteln über einen chemischen Kopplungsschritt mit derartigen Polymeren verbunden werden.

Eine bevorzugte Ausführungsform stellen all solche bislang ausgeführten Proteine oder Derivate dar, die dadurch gekennzeichnet sind, daß sie aus einer natürlichen Quelle, insbesondere aus einem Mikroorganismus erhältlich sind.

Dies können beispielsweise einzellige Pilze oder Bakterien sein. Denn sie lassen sich zumeist einfacher gewinnen und handhaben als vielzellige Organismen oder die von Vielzellern abgeleiteten Zellkulturen; obgleich diese für spezielle Ausführungsformen sinnvolle Optionen darstellen können und somit nicht grundsätzlich vom Erfindungsgegenstand ausgeschlossen sind.

Es ist möglich, daß natürlich vorkommende Produzenten zwar ein erfindungsgemäßes Enzym herstellen können, dieses aber unter den zunächst ermittelten Bedingungen nur in geringem Maße exprimieren und/oder in das umgebende Medium abgeben. Das schließt aber nicht aus, daß geeignete Umweltbedingungen oder sonstige Faktoren experimentell ermittelt werden können, unter deren Einwirken sie zu einer wirtschaftlich sinnvollen Produktion des erfindungsgemäßen Proteins angeregt werden können. Solch ein Regulationsmechanismus kann für die biotechnologische Produktion gezielt eingesetzt werden. Sollte auch dies nicht möglich sein, können sie immer noch der Isolierung des zugehörigen Gens dienen.

Besonders bevorzugt sind hierunter solche aus gram-positiven Bakterien.

Denn diese besitzen keine äußere Membran und geben sekretierte Proteine somit unmittelbar ins umgebende Medium ab.

Ganz besonders bevorzugt sind solche aus gram-positiven Bakterien der Gattung Bacillus.

Bacillus-Proteasen weisen von vornherein günstige Eigenschaften für diverse technische Einsatzmöglichkeiten auf. Dazu gehören eine gewisse Stabilität gegenüber erhöhter Temperatur, oxidierenden oder denaturierenden Agentien. Zudem hat man mit mikrobiellen Proteasen die größten Erfahrungen hinsichtlich ihrer biotechnologischen Produktion, was beispielsweise die Konstruktion günstiger Klonierungsvektoren, die Auswahl von Wirtszellen und Wachstumsbedingungen oder die Abschätzung von Risiken, wie beispielsweise die Allergenizität, angeht. Bacilli sind zudem als Produktionsorganismen mit einer besonders hohen Produktionsleistung in technischen Prozessen etabliert. Der Erfahrungsschatz, den man für die Herstellung und den Einsatz dieser Proteasen erworben hat, kommt zudem erfindungsgemäßen Weiterentwicklungen dieser Enzyme zugute. Dies betrifft beispielsweise ihre Kompatibilität mit anderen chemischen Verbindungen, wie beispielsweise die Inhaltsstoffe von Wasch- oder Reinigungsmitteln.

Unter denen aus den Bacillus-Species, wiederum, sind solche aus der Spezies *Bacillus gibsonii,* insbesondere aus dem Stamm *Bacillus gibsonii* (DSM 14393) bevorzugt.

Denn aus diesem wurde die Ausführungsform des erfindungsgemäßen Enzyms ursprünglich erhalten. Seine zugehörigen Sequenzen sind im Sequenzprotokoll angegeben und seine enzymatischen Charakteristika sind in den Beispielen beschrieben. Von diesem oder von verwandten Stämmen können die oben beschriebenen Varianten insbesondere unter Anwendung molekularbiologischer Standardmethoden, wie beispielsweise der PCR und/oder an sich bekannten Punktmutagenese-Verfahren hergestellt werden.

Eine weitere Lösung der Aufgabe und somit einen eigenen Erfindungsgegenstand stellen die Nukleinsäuren dar, die der Verwirklichung der Erfindung dienen.

Denn Nukleinsäuren bilden den Ausgangspunkt nahezu aller molekularbiologischen Untersuchungen und Weiterentwicklungen sowie der Produktion von Proteinen. Dazu gehören insbesondere die Sequenzierung der Gene und die Ableitung der zugehörigen Aminosäure-Sequenz, jede Art von Mutagenese (siehe oben) und die Expression der Proteine.

Mutagenese zur Entwicklung von Proteinen mit bestimmten Eigenschaften wird auch als "Protein Engineering" bezeichnet. Eigenschaften, auf die optimiert wird, sind weiter oben bereits beispielhaft ausgeführt worden. Solch eine Mutagenese kann zielgerichtet oder über zufallsgemäße Methoden, beispielsweise mit einem anschließenden auf die Aktivität gerichteten Erkennungs- und/oder Auswahlverfahren (Screening und Selektion) an den klonierten Genen, etwa über Hybridisierung mit Nukleinsäure-Sonden, oder an den Genprodukten, den Proteinen, etwa über ihre Aktivität durchgeführt werden. Die Weiterentwicklung der erfindungsgemäßen Proteasen kann insbesondere auch an den in der Publikation "Protein engineering" von P.N.Bryan (2000) in Biochim. Biophys. Acta, Band 1543, S. 203-222, vorgestellten Überlegungen ausgerichtet werden.

Ein Gegenstand der vorliegenden Erfindung sind alle Nukleinsäuren, die für eines der oben beschriebenen, erfindungsgemäßen Proteine oder Derivate codieren.

Die Nukleinsäuren, die für die oben ausgeführten, bevorzugten Formen codieren, sind entsprechend bevorzugt, insbesondere auch die durch Mutagenese erhaltenen Nukleinsäuren.

Insbesondere die Nukleinsäuren, die für Proteinfragmente codieren, werden ausdrücklich in den Schutzbereich der vorliegenden Erfindung einbezogen. Bei solchen Oligonukleotiden sind alle drei Leseraster sowohl in *sense*- als auch in *antisense-*Orientierung zu berücksichtigen. Denn sie können, insbesondere über die Polymerase-Kettenreaktion (PCR) als Ausgangspunkte zur Synthese verwandter Nukleinsäuren verwendet werden, beispielsweise zur Amplifikation verwandter Gene aus natürlichen Organismen. Sie können auch zur Erzeugung von Chimären über ein PCR-basiertes Shuffling-Verfahren dienen. Auch andere Shuffling-Verfahren, wie etwa die Recombining Ligation Reaction (RLR) gemäß der Anmeldung WO 00/09679 beruhen auf Oligonukleotiden, die zufallsgemäß oder zielgerichtet ausgewählten Proteinfragmenten entsprechen. *Antisense*-Oligonukleotide können beispielsweise auch zur Expressions-Regulation eingesetzt werden.

Entsprechend den oben gemachten Angaben sind unter den oben beschriebenen, erfindungsgemäßen Nukleinsäuren folgende zunehmend bevorzugt:
- Solche, die dadurch gekennzeichnet sind, daß sie aus einer natürlichen Quelle, insbesondere aus einem Mikroorganismus erhältlich sind;
- hierunter solche, die dadurch gekennzeichnet sind, daß es sich bei dem Mikroorganismus um ein gram-positives Bakterium handelt;
- hierunter solche, die dadurch gekennzeichnet sind, daß es sich bei dem gram-positiven Bakterium um eines der Gattung Bacillus handelt; und
- hierunter solche, die dadurch gekennzeichnet sind, daß es sich bei der Bacillus-Spezies um *Bacillus gibsonii,* insbesondere um *Bacillus gibsonii* (DSM 14393) handelt.

Einen eigenen Erfindungsgegenstand bilden Vektoren, die einen der zuvor bezeichneten, erfindungsgemäßen Nukleinsäurebereiche enthalten, insbesondere einen, der für eines der zuvor bezeichneten, erfindungsgemäßen Proteine, codiert.

Denn um mit den erfindungsrelevanten Nukleinsäuren umzugehen, und damit insbesondere die Produktion erfindungsgemäßer Proteine vorzubereiten, werden sie geeigneterweise in Vektoren ligiert. Solche Vektoren sowie die zugehörigen Arbeitsmethoden sind im Stand der Technik ausführlich beschrieben. Vektoren sind in großer Zahl und Variationsbreite, sowohl für die Klonierung als auch für die Expression kommerziell erhältlich. Dazu gehören beispielsweise Vektoren, die sich von bakteriellen Plasmiden, von Bacteriophagen oder von Viren ableiten, oder überwiegend synthetische Vektoren. Ferner werden sie nach der Art der Zelltypen, in denen sie sich zu etablieren vermögen, beispielsweise nach Vektoren für gram-negative, für gram-positive Bakterien, für Hefen oder für höhere Eukaryonten unterschieden. Sie bilden geeignete Ausgangspunkte beispielsweise für molekularbiologische und biochemische Untersuchungen sowie für die Expression des betreffenden Gens oder zugehörigen Proteins.

In einer Ausführungsform handelt es sich bei erfindungsgemäßen Vektoren um Klonierungsvektoren.

Denn Klonierungsvektoren eignen sich neben der Lagerung, der biologischen Amplifikation oder der Selektion des interessierenden Gens für dessen molekularbiologische Charakterisierung. Gleichzeitig stellen sie transportierbare und lagerfähige Formen der beanspruchten Nukleinsäuren dar und sind auch Ausgangspunkte für molekularbiologische Techniken, die nicht an Zellen gebunden sind, wie beispielsweise die PCR oder *In*-*vitro*-Mutagenese-Verfahren.

Vorzugsweise handelt es sich bei erfindungsgemäßen Vektoren um Expressionsvektoren.

Denn derartige Expressionsvektoren sind die Basis dafür, die entsprechenden Nukleinsäuren in biologischen Produktionssystemen zu realisieren und damit die zugehörigen Proteine zu produzieren. Bevorzugte Ausführungsformen dieses Erfindungsgegenstands sind Expressionsvektoren, die zur Expression notwendigen genetischen Elemente tragen, beispielsweise den natürlichen, ursprünglich vor diesem Gen lokalisierten Promotor oder einen Promotor aus einem anderen Organismus. Diese Elemente können beispielsweise in Form einer sogenannten Expressionskassette angeordnet sein. Alternativ können einzelne oder alle Regulationselemente auch von der jeweiligen Wirtszelle bereitgestellt werden. Besonders bevorzugt sind die Expressionsvektoren hinsichtlich weiterer Eigenschaften, wie beispielsweise die optimale Kopienzahl, auf das gewählte Expressionssystem, insbesondere die Wirtszelle (siehe unten) abgestimmt.

Vorteilhaft für eine hohe Expressionsrate ist es weiterhin, wenn der Expressionsvektor möglichst nur das betreffende Gen als Insert enthält und keine größeren 5'- oder 3'-nichtcodierenden Bereiche. Solche Inserts werden beispielsweise erhalten, wenn das nach statistischer Behandlung der chromosomalen DNA des Ausgangsstamms mit einem Restriktionsenzym erhaltene Fragment nach der Sequenzierung vor der Integration in den Expressionsvektor noch einmal gezielt geschnitten worden ist.

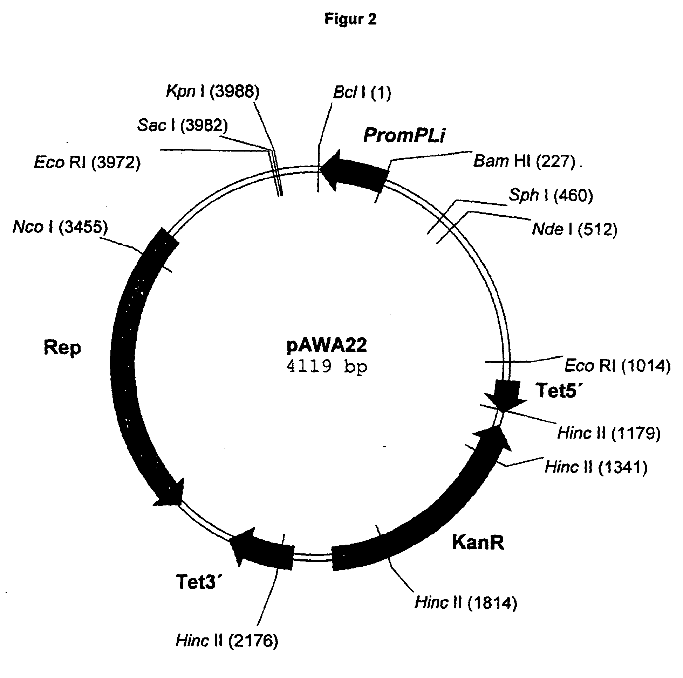

Ein Beispiel für einen Expressionsvektor ist der in Figur 2 der vorliegenden Anmeldung abgebildete und gemäß Beispiel 2 einsetzbare pAWA22. Weitere Vektoren stehen dem Fachmann aus dem Stand der Technik zur Verfügung und werden in großer Zahl kommerziell angeboten.

Einen eigenen Erfindungsgegenstand bilden Zellen, die einen der zuvor bezeichneten, erfindungsgemäßen Nukleinsäurebereiche enthalten, insbesondere einen, der für eines der zuvor bezeichneten, erfindungsgemäßen Proteine oder Derivate codiert, vorzugsweise auf einem der oben bezeichneten, erfindungsgemäßen Vektoren.

Denn diese Zellen enthalten die genetische Information zur Synthese eines erfindungsgemäßen Proteins. Sie ermöglichen beispielsweise die Amplifikation der entsprechenden Gene, aber auch deren Mutagenese oder Transkription und Translation und letztlich die biotechnologische Produktion der betreffenden Proteine. Diese genetische Information kann entweder extrachromosomal als eigenes genetisches Element, das heißt bei Bakterien in plasmidaler Lokalisation vorliegen oder in ein Chromosom integriert sein. Die Wahl eines geeigneten Systems hängt von Fragestellungen, wie beispielsweise die Art und Dauer der Lagerung des Gens, beziehungsweise des Organismus oder die Art der Mutagenese oder Selektion ab. So sind im Stand der Technik beispielsweise auf Bakteriophagen - und deren spezifischen Wirtszellen - beruhende Mutagenese- und Selektionsverfahren zur Entwicklung von Waschmittelenzymen beschrieben (WO 97/09446).

Vorzugsweise handelt es sich dabei um Wirtszellen, die eines der zuvor beschriebenen, erfindungsgemäßen Proteine, Proteinfragmente, Fusionsproteine oder Derivate exprimieren oder zu dessen Expression angeregt werden können, insbesondere unter Einsatz eines der zuvor bezeichneten, erfindungsgemäßen Nukleinsäurebereiche, ganz besonders unter Einsatz eines zuvor bezeichneten Expressionsvektors.

Denn die die Proteine bildenden Wirtszellen ermöglichen deren biotechnologische Produktion. Sie müssen dafür das betreffende Gen, geeigneterweise mit einem der oben beschriebenen Vektoren erhalten haben und zu dessen Transkription, zur Translation und vorzugsweise den eventuell zusätzlichen Modifikationsschritten befähigt sein.

Als Wirtszellen zur Proteinexpression eignen sich prinzipiell alle Organismen, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor, dessen stabile Etablierung und die Regulation der Expression angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Vorzugsweise werden Laborstämme gewählt, die auf die Expression ausgerichtet sind. Solche sind kommerziell oder über allgemein zugängliche Stammsammlungen erhältlich. Jedes erfindungsgemäße Protein kann auf diese Weise theoretisch aus einer Vielzahl von Wirtsorganismen gewonnen werden. Aus der Fülle an verschiedenen nach dem Stand der Technik zur Verfügung stehenden Systeme müssen die optimalen Expressionssysteme für den Einzelfall experimentell ermitteln werden.

Besonders vorteilhaft sind Wirtszellen, die selbst Protease-negativ sind und somit gebildete Proteine nicht abbauen. Um solch einen handelt es sich bei dem in Beispiel 2 verwendeten Stamm *Bacillus subtilis* DB 104.

Bevorzugte Ausführungsformen stellen solche Wirtszellen dar, die aufgrund entsprechender genetischer Elemente in ihrer Aktivität regulierbar sind, beispielsweise durch kontrollierte Zugabe von chemischen Verbindungen, durch Änderung der Kultivierungsbedingungen oder in Abhängigkeit von der jeweiligen Zelldichte. Diese kontrollierbare Expression ermöglicht eine sehr wirtschaftliche Produktion der interessierenden Proteine. Geeigneterweise sind Gen, Expressionsvektor und Wirtszelle aufeinander abgestimmt, was beispielsweise die zur Expression notwendigen genetischen Elemente (Ribosomen-Bindungsstelle, Promotoren, Terminatoren) oder die Codon-Usage betrifft. Letztere kann beispielsweise dadurch optimiert werden, daß in dem Gen jene Codons, die von dem betreffenden Wirt nur schlecht translatiert werden, bei jeweils gleicher Bedeutung durch die für den jeweiligen Wirt gebräuchlicheren ersetzt werden.

Bevorzugt sind darunter Wirtszellen, die dadurch gekennzeichnet sind, daß sie Bakterien sind, insbesondere solche, die das gebildete Protein ins umgebende Medium sekretieren.

Denn Bakterien zeichnen sich durch kurze Generationszeiten und geringe Ansprüche an die Kultivierungsbedingungen aus. Dadurch können kostengünstige Verfahren etabliert werden. Zudem verfügt man bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf etc. gram-negative oder gram-positive Bakterien geeignet sein.

Bei gram-negativen Bakterien, wie beispielsweise *E. coli,* werden eine Vielzahl von Proteinen in den periplasmatischen Raum sekretiert. Dies kann für spezielle Anwendungen vorteilhaft sein. Gram-positive Bakterien, wie beispielsweise Bacilli, geben demgegenüber sekretierte Proteine sogleich in das die Zellen umgebende Nährmedium ab, aus welchem sich nach einer anderen bevorzugten Ausführungsform die exprimierten erfindungsgemäßen Proteine direkt aufreinigen lassen.

In der Anmeldung WO 01/81597 wird ein Verfahren offenbart, nach welchem erreicht wird, daß auch gram-negative Bakterien die exprimierten Proteine ausschleusen. Solch ein System ist auch für die Herstellung erfindungsgemäßer Proteine geeignet. Als Wirtszellen sind demnach solche der Spezies *Escherichia coli* oder *Klebsiella* bevorzugt, besonders solche der Stämme *E*. *coli* JM 109, *E*. *coli* DH 100B, *E*. *coli* DH 12S oder *Klebsiella planticola* (Rf). Sie erfordern entsprechende mikrobiologische Modifikationen und/oder geeignete, in dieser Anmeldung beschriebene Vektoren, um die Freisetzung der produzierten Proteine zu ermöglichen.

Als Wirtszellen bevorzugte Bakterien sind solche, die dadurch gekennzeichnet sind, daß sie gram-positive Bakterien sind, insbesondere daß sie der Gattung Bacillus angehören, ganz besonders der Species *Bacillus lentus, Bacillus licheniformis, Bacillus amyloliquefaciens, Bacillus subtilis* oder *Bacillus alcalophilus.*

Eine Ausführungsform der vorliegenden Erfindung nutzt *B. gibsonii,* insbesondere *B. gibsonii* (DSM 14393) selbst, um erfindungsgemäße Proteine (homolog) zu exprimieren. Demgegenüber ist jedoch die heterologe Expression bevorzugt. Hierfür werden Bakterien der Gattung Bacillus bevorzugt, weil sie unter den gram-positiven Bakterien produktionsstechnisch am besten charakterisiert sind. Hierzu gehören insbesondere solche der Species *B. licheniformis, B. amyloliquefaciens, B. subtilis* oder andere Species oder Stämme von *B. alcalophilus.* Denn mit diesen Spezies hat man, beispielsweise über die Lehre der Anmeldung WO 91/02792, einschlägige Erfahrungen, was die Protease-Herstellung angeht. In dieser Anmeldung sind auch zahlreiche mögliche Expressionsvektoren offenbart. Diese verwandten Species verfügen zudem über eine ähnliche Codon-Usage und bilden vergleichbare Subtilisine selbst, so daß ihr Protein-Syntheseapparat naturgemäß entsprechend ausgerichtet ist.

Ein weiterer Vorteil besteht darin, daß über dieses Verfahren eine Mischung erfindungsmäßer Proteine mit den endogen von den Wirtsstämmen gebildeten Subtilisinen erhalten werden kann. Solch eine Coexpression geht ebenfalls aus der Anmeldung WO 91/02792 hervor. Sollte sie nicht gewünscht sein, müßten die in der Wirtszelle natürlicherweise vorhandenen Proteasegene dauerhaft oder vorübergehend inaktiviert werden (siehe oben).

Weiter bevorzugt sind Wirtszellen, die dadurch gekennzeichnet sind, daß sie eukaryontische Zellen sind, insbesondere solche, die das gebildete Protein posttranslational modifizieren.

Beispiele für geeignete Eukaryonten sind Pilze wie Actinomyceten oder Hefen wie *Saccharomyces* oder *Kluyveromyces.* Thermophile pilzliche Expressionssysteme werden beispielsweise in WO 96/02653 vorgestellt. Solche eignen sich besonders zur Expression temperaturbeständiger Varianten. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität wünschenswert sein. Auch eine Coexpression mit den natürlicherweise von derartigen Zellen gebildeten Enzymen, wie beispielsweise Cellulasen, kann vorteilhaft sein.

Einen eigenständigen Erfindungsgegenstand stellen Verfahren zur Herstellung eines erfindungsgemäßen Proteins dar.

Beansprucht wird somit jedes Verfahren zur Herstellung eines oben beschriebenen, erfindungsgemäßen Proteins, Proteinfragments, Fusionsproteins oder Derivats unter Einsatz einer oben beschriebenen, erfindungsgemäßen Nukleinsäure und/oder unter Einsatz eines oben beschriebenen, erfindungsgemäßen Vektors und/oder unter Einsatz einer der oben beschriebenen, erfindungsgemäßen Zellen.

Hierzu gehören beispielsweise chemische Syntheseverfahren, die insbesondere für kürzere Fragmente wirtschaftlich sinnvoll sind.

Demgegenüber bevorzugt sind jedoch alle im Stand der Technik etablierten, oben in einzelnen Aspekten bereits angesprochenen molekularbiologischen, mikrobiologischen, beziehungsweise biotechnologischen Herstellverfahren. Demnach können beispielsweise anhand der oben bezeichneten DNA- und Aminosäuresequenzen, wie sie beispielsweise auch aus dem Sequenzprotokoll abgeleitet werden können, vorzugsweise anhand derer aus SEQ ID NO. 1 und 2 selbst, entsprechende Oligonukleotide und Oligopeptide bis hin zu den vollständigen Genen und Proteinen nach an sich bekannten molekularbiologischen Methoden synthetisiert werden.

Ausgehend von den bekannten Subtilisin-produzierenden Mikroorganismen können, beispielsweise in Anlehnung an die Beispiel in der vorliegenden Anmeldung, auch weitere natürliche Produzenten von Subtilisinen identifiziert und isoliert werden, deren Subtilisin-Gen- und/oder Aminosäuresequenzen ermittelt und entsprechend der hier gemachten Vorgaben weiterentwickelt werden. Solche Bakterienspezies können auch für entsprechende Herstellungsverfahren kultiviert und eingesetzt werden. Analog können nach dem Vorbild der beispielsweise in der Anmeldung WO 91/02792 offenbarten Vektoren neue Expressionsvektoren entwickelt werden. Ausführungsformen der vorliegenden Erfindung können auf der Grundlage der zugehörigen Nukleinsäuresequenzen auch zellfreie Expressionssysteme sein, bei denen die Proteinbiosynthese *in vitro* nachvollzogen wird. Alle bereits oben ausgeführten Elemente können auch zu neuen Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar, so daß optimale Verfahren für jeden konkreten Einzelfall experimentell ermittelt werden müssen.

Einen eigenen Erfindungsgegenstand stellen Mittel dar, die dadurch gekennzeichnet sind, daß sie ein oben beschriebenes, erfindungsgemäßes Protein oder Derivat enthalten.

Alle Arten von Mitteln, insbesondere Gemische, Rezepturen, Lösungen etc., deren Einsetzbarkeit durch Zugabe eines oben beschriebenen, erfindungsgemäßen Proteins verbessert wird, werden hiermit in den Schutzbereich der vorliegenden Erfindung eingeschlossen. Es kann sich dabei je nach Einsatzgebiet beispielsweise um feste Gemische, beispielsweise Pulver mit gefriergetrockeneten oder verkapselten Proteinen, oder um gelförmige oder flüssige Mittel handeln. Bevorzugte Rezepturen enthalten beispielsweise Puffersubstanzen, Stabilisatoren, Reaktionspartner und/oder Cofaktoren der Proteasen und/oder andere mit den Proteasen synergistische Inhaltsstoffe. Insbesondere sind darunter Mittel für die weiter unten ausgeführten Einsatzgebiete zu verstehen. Weitere Einsatzgebiete gehen aus dem Stand der Technik hervor und werden beispielsweise in dem Handbuch "Industrial enyzmes and their applications" von H. Uhlig, Wiley-Verlag, New York, 1998 dargestellt,

Diesem Erfindungsgegenstand werden als bevorzugte Ausführungsform Wasch- oder Reinigungsmittel zugerechnet, die dadurch gekennzeichnet sind, daß sie eines der zuvor beschriebenen, erfindungsgemäßen Proteine oder Derivate enthalten.

Denn wie in den Ausführungsbeispielen der vorliegenden Anmeldung gezeigt ist, konnte überraschenderweise festgestellt werden, daß sich die besonders bevorzugte Alkalische Protease aus *B. gibsonii* (DSM 14393), das heißt bereits das Wildtyp-Enzym dadurch auszeichnet, daß es bei Verwendung in einem entprechenden Wasch- oder Reinigungsmittel den für diesen Zweck etablierten Enzymen in ihren Beiträgen zur Wasch-, beziehungsweise Reinigungsleistung mindestens nahekommt oder sie teilweise sogar übertrifft.

Zu diesem Erfindungsgegenstand zählen alle denkbaren Reinigungsmittelarten, sowohl Konzentrate als auch unverdünnt anzuwendende Mittel, zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung **Waschmittel** verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung **Reinigungsmittel** verwendet. Jegliche Wasch- oder Reinigungsmittelart stellt eine Ausführungsform der vorliegenden Erfindung dar, sofern sie um ein erfindungsgemäßes Protein, Proteinfragment, Fusionsprotein oder Derivat bereichert ist.

Ausführungsformen der vorliegenden Erfindung umfassen alle nach den Stand der Technik etablierten und/oder alle zweckmäßigen Darreichungsformen der erfindungsgemäßen Wasch- oder Reinigungsmittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

In einer bevorzugten Ausführungsform enthalten die erfindungsgemäßen Wasch- oder Reinigungsmittel die oben beschriebenen, erfindungsgemäßen Proteine oder Derivate in einer Menge von 2 µg bis 480 mg, vorzugsweise von 5 µg bis 420 mg, besonders bevorzugt von 20 µg bis 360 mg, ganz besonders bevorzugt von 50 µg bis 240 mg pro Gramm des Mittels.

Die Proteaseaktivität in derartigen Mitteln kann nach der in Tenside, Band 7 (1970), S. 125-132 beschriebenen Methode ermittelt werden. Sie wird dementsprechend in PE (Protease-Einheiten) angegeben.

Neben einem erfindungsgemäßen Protein oder Derivat enthält ein erfindungsgemäßes Wasch- oder Reinigungsmittel gegebenenfalls weitere Inhaltsstoffe wie Enzymstabilisatoren, Tenside, z. B. nichtionische, anionische und/oder amphotere Tenside, und/oder Bleichmittel, und/oder Builder, sowie gegebenenfalls weitere übliche Inhaltsstoffe

Zu den für Wasch- und Reinigungsmitteln üblichen Inhaltsstoffen werden im allgemeinen auch wasch-, beziehungsweise reinigungsaktive Enzyme gerechnet.

Somit stellen Wasch- oder Reinigungsmittel, die über ein oben beschriebenes, erfindungsgemäßes Protein oder Derivat hinaus durch zusätzlich weitere Enzyme gekennzeichnet sind, bevorzugte Ausführungsformen der vorliegenden Erfindung dar. Dazu gehören insbesondere andere Proteasen, Amylasen, Cellulasen, Hemicellulasen wie beispielsweise β-Glucanasen. Oxidoreductasen wie beispielsweise Laccasen, Cutinasen und/oder Lipasen, aber auch Esterasen und alle anderen Enzyme, die aus dem Stand der Technik für dieses Einsatzgebiet beschrieben sind.

Enzyme wie Proteasen, Amylasen, Lipasen oder Cellulasen werden seit Jahrzehnten als aktive Komponenten in Wasch- und Reinigungsmitteln verwendet. Ihr jeweiliger Beitrag zur Wasch-, beziehungsweise Reinigungsleistung des betreffenden Mittels ist im Fall von Protease die Fähigkeit zum Abbau proteinhaltiger Verunreinigungen, im Fall von Amylase der Abbau von stärkehaltigen Verunreinigungen und im Fall von Lipase die fettspaltende Aktivität. Cellulasen werden über ihre schmutzentfemende, das heißt primäre Wasch-, beziehungsweise Reinigungsleistung hinaus, insbesondere wegen ihres Beitrags zur Sekundärwaschleistung eines Waschmittels und wegen ihrer Faserwirkung auf Textilien bevorzugt in Waschmitteln eingesetzt. Die jeweiligen Hydrolyseprodukte werden von den übrigen Wasch- oder Reinigungsmittel-Bestandteilen angegriffen, gelöst, emulgiert oder suspendiert oder aufgrund ihrer höheren Löslichkeit mit der Waschflotte ausgeschwemmt, so daß es günstigenfalls zu Synergieeffekten zwischen den Enzymen und den übrigen Bestandteilen kommt.

Einen dem Beitrag zur Sekundärwaschleistung eines Waschmittels durch Cellulase vergleichbaren Effekt können Proteasen auf natürliche Fasern, insbesondere auf Wolle oder Seide ausüben. Durch ihre Wirkung auf die Oberflächenstruktur solcher Gewebe können sie einen glättenden Einfluß auf das Material ausüben und damit dem Verfilzen entgegenwirken.

Weitere Enzyme erweitern die Reinigungsleistung entsprechender Mittel um ihre jeweils spezifische enzymatische Leistung. Dazu gehören beispielsweise Hemicellulasen wie beispielsweise β-Glucanasen (WO 99/06515 und WO 99/06516), Oxidoreduktasen wie zum Beispiel Laccasen (WO 00/39306) oder Pektin-lösende Enzyme (WO 00/42145), die insbesondere in Speziatwaschmitteln zum Einsatz kommen.

Zur Verwendung in erfindungsgemäßen Wasch- oder Reinigungsmitteln kommen in erster Linie aus Mikroorganismen, wie Bakterien oder Pilzen, gewonnene Enzyme in Frage. Sie werden in bekannter Weise durch Fermentationsprozesse aus geeigneten Mikroorganismen gewonnen, die zum Beispiel in den deutschen Offenlegungsschriften DE 1940488, und DE 2121397, den US-amerikanischen Patentschriften US 3623957, US 4264738, der europäischen Patentanmeldung EP 006638 sowie der internationalen Patentanmeldung WO 91/02792 beschrieben sind.

Ein erfindungsgemäßes Protein und/oder andere enthaltene Proteine können besonders während der Lagerung durch Stabilisatoren beispielsweise vor Denaturierung, Zerfall oder Inaktivierung, etwa durch physikalische Einflüsse, Oxidation oder proteolytische Spaltung geschützt werden. Dies gilt für alle erfindungsgemäßen Mittel, insbesondere Wasch- und Reinigungsmittel.

Eine Gruppe von Stabilisatoren sind reversible Proteaseinhibitoren, die bei Verdünnung des Mittels in der Waschflotte abdissozieren. Benzamidin-Hydrochlorid und Leupeptin sind für diesen Zweck etabliert. Häufig werden Borax, Borsäuren, Boronsäuren oder deren Salze oder Ester verwendet, darunter vor allem Derivate mit aromatischen Gruppen, etwa gemäß WO 95/12655 ortho-substituierte, gemäß WO 92/19707 metasubstituierte und gemäß US 5972873 para-substituierte Phenylboronsäuren, beziehungsweise deren Salze oder Ester. In den Anmeldungen WO 98/13460 und EP 583534 werden Peptidaldehyde, das heißt Oligopeptide mit reduziertem C-Terminus, und zwar solche aus 2-50 Monomeren, zur reversiblen Inhibierung von Wasch- und Reinigungsmittelproteasen offenbart. Zu den peptidischen reversiblen Proteaseinhibitoren gehört u.a. Ovomucoid (WO 93/00418). Beispielsweise mit der Anmeldung WO 00/01826 werden spezifische, reversible Peptid-Inhibitoren für die Protease Subtilisin zur Verwendung in Protease-haltigen Mitteln offenbart, und mit WO 00/01831 entsprechende Fusionsproteine aus Protease und Inhibitor.

Weitere Enzymstabilisatoren sind Aminoalkohole wie Mono-, Di-, Triethanol- und -propanolamin und deren Mischungen, aliphatische Carbonsäuren bis zu C₁₂, wie beispielsweise aus den Anmeldungen EP 378261 und WO 97/05227 bekannt ist, wie Bernsteinsäure, andere Dicarbonsäuren oder Salze der genannten Säuren. Mit der Anmeldung DE 19650537 werden für diesen Zweck endgruppenverschlossene Fettsäureamidalkoxylate offenbart. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Ebenso werden Calciumsalze verwendet, wie beispielsweise Calciumacetat oder das in der Patentschrift EP 028865 für diesen Zweck offenbarte Calcium-Formiat, und Magnesiumsalze, etwa gemäß der europäischen Anmeldung EP 378262.

Polyamid-Oligomere (WO 99/43780) oder polymere Verbindungen wie Lignin (WO 97/00932), wasserlösliche Vinyl-Copolymere (EP 828762) oder, wie in EP 702712 offenbart, Cellulose-Ether, Acryl-Polymere und/oder Polyamide stabilisieren die Enzym-Präparation u. a. gegenüber physikalischen Einflüssen oder pH-Wert-Schwankungen. Polyamin-N-Oxid-enthaltende Polymere (EP 587550 und EP 581751) wirken gleichzeitig als Enzymstabilisatoren und als Farbübertragungsinhibitoren. Andere polymere Stabilisatoren sind die in WO 97/05227 neben anderen Bestandteilen offenbarten linearen C₈-C₁₈ Polyoxyalkylene. Alkylpolyglycoside könnten wie in den Anmeldungen WO 97/43377 und WO 98/45396 die enzymatischen Komponenten des erfindungsgemäßen Mittels stabilisieren und sogar in ihrer Leistung steigern. Vernetzte N-haitige Verbindungen, wie in WO 98/17764 offenbart, erfüllen eine Doppelfunktion als Soil-release-Agentien und als Enzym-Stabilisatoren. Hydrophobes, nichtionisches Polymer wirkt in einer Mischung mit anderen Stabilisatoren gemäß der Anmeldung WO 97/32958 stabilisierend auf eine Cellulase, so daß sich solche oder ähnliche Bestandteile auch für das erfindungswesentliche Enzym eignen könnten.

Reduktionsmittel und Antioxidantien erhöhen, wie u.a. in EP 780466 offenbart, die Stabilität der Enzyme gegenüber oxidativem Zerfall. Schwefelhaltige Reduktionsmittel sind beispielsweise aus den Patentschriften EP 080748 und EP 080223 bekannt. Andere Beispiele sind Natrium-Sulfit (EP 533239) und reduzierende Zucker (EP 656058).

Vielfach werden auch Kombinatonen von Stabilisatoren verwendet, beispielsweise aus Polyolen, Borsäure und/oder Borax in der Anmeldung WO 96/31589, die Kombination von Borsäure oder Borat, reduzierenden Salzen und Bernsteinsäure oder anderen Dicarbonsäuren in der Anmeldung EP 126505 oder die Kombination von Borsäure oder Borat mit Polyolen oder Polyaminoverbindungen und mit reduzierenden Salzen, wie in der Anmeldung EP 080223 offenbart. Die Wirkung von Peptid-Aldehyd-Stabilisatoren wird gemäß WO 98/13462 durch die Kombination mit Borsäure und/oder Borsäurederivaten und Polyolen gesteigert und gemäß WO 98/13459 durch die zusätzliche Verwendung von Calcium-lonen noch weiter verstärkt.

Mittel mit stabilisierten Enzymaktivitäten stellen bevorzugte Ausführungsformen der vorliegenden Erfindung dar. Besonders bevorzugt sind solche mit Enzymen, die auf mehrere der dargestellten Weisen stabilisiert sind.

Da erfindungsgemäße Mittel in allen denkbaren Formen angeboten werden können, stellen erfindungsgemäße Enzyme, beziehungsweise Proteine in allen für die Zugabe zu den jeweiligen Mitteln zweckmäßigen Formulierungen jeweilige Ausführungsformen der vorliegenden Erfindung dar. Dazu gehören beispielsweise flüssige Formulierungen, feste Granulate oder Kapseln.

Eine weitere Ausführungsform stellen Mittel zur Behandlung von Textilrohstoffen oder zur Textilpflege dar, die dadurch gekennzeichnet sind, daß sie allein oder neben anderen aktiven Inhaltsstoffen eines der oben beschriebenen, erfindungsgemäßen Proteine oder Derivate enthalten, insbesondere für Fasern oder Textilien mit natürlichen Bestandteilen und ganz besonders für solche mit Wolle oder Seide.

Denn insbesondere natürliche Fasern, wie beispielsweise Wolle oder Seide, zeichnen sich durch eine charakteristische, mikroskopische Oberflächenstruktur aus. Diese kann, wie am Beispiel der Wolle im Artikel von R. Breier in Melliand Textilberichte vom 1.4.2000 (S. 263) ausgeführt worden ist, langfristig zu unerwünschten Effekten, wie etwa Verfilzung führen. Zur Vermeidung solcher Effekte werden die natürlichen Rohstoffe mit erfindungsgemäßen Mitteln behandelt, welche beispielsweise dazu beitragen, die auf Proteinstrukturen beruhende geschuppte Oberflächenstruktur zu glätten und damit einem Verfilzen entgegenwirken.

In einer bevorzugten Ausführungsform ist das Mittel mit einer erfindungsgemäßen Protease so konzipiert, daß es regelmäßig als Pflegemittel verwendet werden kann, beispielsweise indem es dem Waschprozeß zugesetzt, nach dem Waschen angewendet oder unabhängig von dem Waschen appliziert wird. Der gewünschte Effekt besteht darin, eine glatte Oberflächenstruktur des Textils über einen langen Zeitraum zu erhalten und/oder Schädigungen des Gewebes vorzubeugen und/oder zu verringern.

Einen eigenen Erfindungsgegenstand stellen Verfahren zur maschinellen Reinigung von Textilien oder von harten Oberflächen dar, die dadurch gekennzeichnet sind, daß in wenigstens einem der Verfahrensschritte ein oben beschriebenes, erfindungsgemäßes Protein Derivat aktiv wird, insbesondere in einer Menge von 40 µg bis 96 g, vorzugsweise von 50 µg bis 72 g, besonders bevorzugt von 100 µg bis 48 g und ganz besonders bevorzugt von 200 µg bis 24 g pro Anwendung. Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind.

Verfahren zur Reinigung von Textilien zeichnen sich im allgemeinen dadurch aus, daß in mehreren Verfahrensschritten verschiedene reinigungsaktive Substanzen auf das Reinigungsgut aufgebracht und nach der Einwirkzeit abgewaschen werden, oder daß das Reinigungsgut in sonstiger Weise mit einem Waschmittel oder einer Lösung dieses Mittels behandelt wird. Das gleiche gilt für Verfahren zur Reinigung von allen anderen Materialien als Textilien, welche unter dem Begriff harte Oberflächen zusammengefaßt werden. Alle denkbaren Wasch- oder Reinigungsverfahren können in wenigstens einem der Verfahrensschritte um erfindungsgemäße Proteine bereichert werden, und stellen dann Ausführungsformen der vorliegenden Erfindung dar.

Da bevorzugte erfindungsgemäße Enzyme natürlicherweise bereits eine proteinauflösende Aktivität besitzen und diese auch in Medien entfalten, die sonst keine Reinigungskraft besitzen, wie beispielsweise in bloßem Puffer, kann ein einzelner Teilschritt eines solchen Verfahrens zur maschinellen Reinigung von Textilien darin bestehen, daß gewünschtenfalls neben stabilisierenden Verbindungen, Salzen oder Puffersubstanzen als einzige reinigungsaktive Komponente ein erfindungsgemäßes Enzym aufgebracht wird. Dies stellt eine besonders bevorzugte Ausführungsform der vorliegenden Erfindung dar.

In einer weiteren bevorzugten Ausführungsform solcher Verfahren werden die betreffenden erfindungsgemäßen Enzyme im Rahmen einer der oben ausgeführten Rezepturen für erfindungsgemäße Mittel, vorzugsweise erfindungsgemäße Wasch-, beziehungsweise Reinigungsmittel bereitgestellt.

Bevorzugte Ausführungsformen dieses Erfindungsgegenstand stellen Verfahren zur Behandlung von Textilrohstoffen oder zur Textilpflege dar, die dadurch gekennzeichnet sind, daß in wenigstens einem der Verfahrensschritte ein oben beschriebenes, erfindungsgemäßes Protein oder Derivat aktiv wird, insbesondere für Textilrohstoffe, Fasern oder Textilien mit natürlichen Bestandteilen und ganz besonders für solche mit Wolle oder Seide.

Es kann sich dabei beispielsweise um Verfahren handeln, in denen Materialien zur Verarbeitung in Textilien vorbereitet werden, etwa zur Antifilzausrüstung, oder beispielsweise um Verfahren, welche die Reinigung getragener Textilien um eine pflegende Komponente bereichern. Wegen der oben beschriebenen Wirkung von Proteasen auf natürliche, proteinhaltige Rohstoffe handelt es sich in bevorzugten Ausführungsformen um Verfahren zur Behandlung von Textilrohstoffen, Fasern oder Textilien mit natürlichen Bestandteilen, insbesondere mit Wolle oder Seide.

Einen eigenen Erfindungsgegenstand stellt die Verwendung eines oben beschriebenen, erfindungsgemäßen Proteins oder Derivats zur Reinigung von Textilien oder von harten Oberflächen dar.

Vorzugsweise gelten für diese Verwendung die oben ausgeführten Konzentrationsbereiche.

Denn erfindungsgemäße Proteine können, insbesondere entsprechend den oben beschriebenen Eigenschaften und den oben beschriebenen Verfahren dazu verwendet werden, um von Textilien oder von harten Oberflächen proteinhaltige Verunreinigungen zu beseitigen. Ausführungsformen stellen beispielsweise die Handwäsche oder die manuelle Entfernung von Flecken von Textilien oder von harten Oberflächen oder die Verwendung im Zusammenhang mit einem maschinellen Verfahren.

In einer bevorzugten Ausführungsform dieser Verwendung werden die betreffenden erfindungsgemäßen Enzyme im Rahmen einer der oben ausgeführten Rezepturen für erfindungsgemäße Mittel, vorzugsweise Wasch-, beziehungsweise Reinigungsmittel bereitgestellt.

Eine weitere Ausführungsform dieses Erfindungsgegenstands stellt die Verwendung eines oben beschriebenen, erfindungsgemäßen Proteins, oder Derivats zur Aktivierung oder Deaktivierung von Inhaltsstoffen von Wasch- oder Reinigungsmitteln dar.

Denn, wie bekannt ist, können Protein-Bestandteile von Wasch- oder Reinigungsmitteln durch das Einwirken einer Protease inaktiviert werden. Diesen ansonsten eher unerwünschten Effekt gezielt einzusetzen, ist ein Gegenstand der vorliegenden Erfindung. Ebenso ist es, wie oben beschrieben, möglich, daß durch Proteolyse eine andere Komponente erst aktiviert wird, etwa, wenn sie ein Hybridprotein aus dem eigentlichen Enzym und dem dazu passenden Inhibitor darstellt, wie dies beispielsweise in der Anmeldung WO 00/01831 offenbart worden ist. Ein anderes Beispiel für eine solche Regulation ist die, bei der eine aktive Komponente zum Schutz oder zur Kontrolle seiner Aktivität in einem Material verkapselt vorliegt, das durch Proteolyse angegriffen wird. Erfindungsgemäße Proteine können somit zu Inaktivierungs-, Aktivierungs- oder Freisetzungsreaktionen verwendet werden, insbesondere in mehrphasigen Mitteln.

Im folgenden werden allen anderen, außerhalb der Wasch- und Reinigungsproblematik angesiedelten technischen Verfahren, Verwendungen und zugehörigen Mittel ungeachtet ihrer Diversität zu einem Erfindungsgegenstand zusammengefaßt, sofern sie durch ein erfindungsgemäßes Protein gekennzeichnet sind. Diese Zusammenstellung ist nicht als abschließende Aufzählung zu verstehen, sondern stellt die wichtigsten, derzeit zu erkennenden Einsatzmöglichkeiten erfindungsgemäßer Proteasen zusammen Anhaltspunkt für weitere, ebenfalls eingeschlossene Einsatzmöglichkeiten bietet beispielsweise auch das Hanbuch "Industrial enyzmes and their applications" von H. Uhlig, Wiley-Verlag, New York, 1998. Sollte sich herausstellen, daß weitere technische Gebiete durch den Einsatz erfindungsgemäßer Proteasen weiterentwickelt werden können, so sind diese in den Schutzbereich der vorliegenden Erfindung eingeschlossen.

Eine Ausführungsform dieses Erfindungsgegenstands stellt die Verwendung eines oben beschriebenen, erfindungsgemäßen Proteins oder Derivats zur biochemischen Analyse oder zur Synthese von niedermolekularen Verbindungen oder von Proteinen dar.

Vorzugsweise erfolgt diese Verwendung im Rahmen entsprechender Mittel oder Verfahren. Erfindungsgemäß und nach Römpp, "Lexikon Chemie" (Version 2.0, Stuttgart/ New York: Georg Thieme Verlag, 1999) wird unter der enzymatischen Analyse jede biochemische Analytik verstanden, die sich spezifischer Enzyme oder Substrate bedient, um einerseits Substrate in ihrer Identität oder ihrer Konzentration oder andererseits Enzyme in Identität oder Aktivität zu bestimmen. Anwendungsgebiete sind alle mit der Biochemie verwandten Arbeitsgebiete, insbesondere die Molekularbiologie und die Proteinchemie. Diese Verwendung erfolgt bevorzugt im Rahmen eines enzymatischen Analyseverfahrens. Eine bevorzugte Ausführungsform dieses Erfindungsgegenstands stellt die Verwendung zur Endgruppenbestimmung im Rahmen einer Sequenzanalyse dar.

Eine weitere Ausführungsform dieses Erfindungsgegenstands stellt die Verwendung eines oben beschriebenen, erfindungsgemäßen Proteins oder Derivats zur Präparation, Reinigung oder Synthese von Naturstoffen oder biologischen Wertstoffen dar.

Vorzugsweise erfolgt diese Verwendung im Rahmen entsprechender Mittel oder Verfahren. So kann es im Verlauf der Aufreinigung von Naturstoffen oder biologischen Wertstoffen beispielsweise notwendig sein, diese von Proteinverunreinigungen zu befreien. Dabei kann es sich beispielweise um niedermolekulare Verbindungen, um alle Zellinhalts- oder Speicherstoffe oder um Proteine handeln. Dies ist sowohl im Labormaßstab, als auch in großtechnischer Dimension, beispielsweise nach der biotechnologischen Herstellung eines Wertstoffes durchführbar.

Die Verwendung eines erfindungsgemäßen proteolytischen Enzyms zur Synthese von Proteinen oder anderen niedermolekularen chemischen Verbindungen geschieht in Umkehrung der von ihnen natürlicherweise katalysierten Reaktion, beispielsweise dann, wenn Proteinfragmente miteinander verknüpft werden oder an eine nicht überwiegend aus Protein bestehende Verbindung Aminosäuren gebunden werden sollen. Derartige Verwendungsmöglichkeiten sind beispielsweise in Anlehnung an die Anmeldung EP 380362 möglich.

Eine weitere Ausführungsform dieses Erfindungsgegenstands stellt die Verwendung eines oben beschriebenen, erfindungsgemäßen Proteins. oder Derivats zur Behandlung von natürlichen Rohstoffen dar, insbesondere zur Oberflächenbehandlung, ganz besonders in einem Verfahren zur Behandlung von Leder.

Vorzugsweise erfolgt diese Verwendung im Rahmen entsprechender Mittel oder Verfahren. Sie ist beispielsweise dann erforderlich, wenn natürliche Rohstoffe von Protein-Verunreinigungen befreit werden sollen. Hierunter sind in erster Linie nichtmikrobiologisch zu erhaltende Rohstoffe, etwa aus der Landwirtschaft, aber auch biotechnologisch über Fermentationen hergestellte Stoffe wie beispielsweise Antibiotika zu verstehen.

Eine bevorzugte Ausführungsform stellt die Verwendung zur Oberflächenbehandlung, und ganz besonders in einem Verfahren zur Behandlung des wirtschaftlich bedeutenden Rohstoffs Leder dar. So werden im Verlauf des Gerbverfahrens, inbesondere im Schritt der Alkalischen Weiche (Römpp, "Lexikon Chemie", Version 2.0, Stuttgart/ New York: Georg Thieme Verlag, 1999) wasserlösliche Proteine mithilfe proteolytischer Enzyme aus dem Hautmaterial herausgelöst. Hierfür eignen sich, insbesondere wenn alkalische Bedingungen herrschen und/oder denaturierende Agentien anwesend sind, erfindungsgemäße Proteasen.

Eine weitere Ausführungsform dieses Erfindungsgegenstands stellt die Verwendung eines oben beschriebenen, erfindungsgemäßen Proteins_{.} oder Derivats zur Gewinnung oder Behandlung von Rohmaterialien oder Zwischenprodukten in der Textilherstellung, insbesondere zum Entfernen von Schutzschichten auf Geweben dar.

Vorzugsweise erfolgt diese Verwendung im Rahmen entsprechender Mittel oder Verfahren dar. Ein Beispiel für die Gewinnung oder Behandlung von Rohmaterialien oder Zwischenprodukten in der Textilherstellung ist die Aufarbeitung von Baumwolle, die in einem als Schlichten bezeichneten Prozeß von den Kapselbestandteilen befreit werden muß; ein anderes die Behandlung von Wolle; ähnlich gestaltet sich auch die Verarbeitung von Rohseide. Enzymatische Verfahren, beziehungsweise Verwendungen sind vergleichbaren chemischen Verfahren besonders hinsichtlich ihrer Umweltverträglickeit überlegen.

In einer bevorzugten Ausführungsform werden erfindungsgemäße Proteine dazu verwendet, um Schutzschichten von Textilien, insbesondere Zwischenprodukten oder Werkstoffen zu entfernen oder ihre Oberfläche zu glätten, bevor sie in einem nachfolgenden Verarbeitungsschritt weiterbearbeitet werden.

Eine weitere Ausführungsform dieses Erfindungsgegenstands stellt die Verwendung eines oben beschriebenen, erfindungsgemäßen Proteins oder Derivats zur Behandlung von Textilrohstoffen oder zur Textilpflege dar, insbesondere zur Behandlung von Wolle oder Seide oder woll- oder seidenhaltigen Mischtextilien.

Vorzugsweise erfolgt diese Verwendung im Rahmen entsprechender Mittel oder Verfahren. Entsprechend dem oben Gesagten werden die betreffenden Textilrohstoffe durch die Protease von Verunreinigungen befreit; außerdem kommen einem wenigstens zum Teil aus Protein bestehenden Material die oberflächenglättenden und pflegenden Eigenschaften des proteolytischen Enzyms zugute. Aus diesem Grunde ist auch die Verwendung zur Pflege der betreffenden Materialien umfaßt. Insbesondere wird deshalb die Oberflächenbehandlung von Wolle oder Seide oder woll- oder seidenhaltigen Mischtextilien beansprucht. Dies gilt sowohl für die Herstellung für derartige Textilien, als auch für die Pflege während des Gebrauchs, beispielsweise im Zusammenhang mit der Textilreinigung (siehe oben).

Eine weitere Ausführungsform dieses Erfindungsgegenstands stellt die Verwendung eines oben beschriebenen, erfindungsgemäßen Proteins oder Derivats zur Behandlung von photographischen Filmen dar, insbesondere zur Entfernung von gelatinhaltigen oder ähnlichen Schutzschichten.

Vorzugsweise erfolgt diese Verwendung im Rahmen entsprechender Mittel oder Verfahren. Denn mit solchen Schutzschichten, insbesondere solchen aus Silbersalzhaltigen Gelatin-Emulsionen werden Filme, wie beispielsweise Röntgenfilme beschichtet. Diese Schichten müssen nach der Belichtung vom Trägermaterial abgelöst werden. Hierfür können insbesondere unter alkalischen oder leicht denaturierenden Reaktionsbedingungen erfindungsgemäße Proteasen verwendet werden.

Eine weitere Ausführungsform dieses Erfindungsgegenstands stellt die Verwendung eines oben beschriebenen, erfindungsgemäßen Proteins oder Derivats zur Herstellung von Lebensmitteln oder von Futtermitteln dar.

Vorzugsweise erfolgt diese Verwendung im Rahmen entsprechender Mittel oder Verfahren. So werden Proteasen schon von alters her zur Lebensmittelherstellung verwendet. Ein Beispiel dafür ist die Verwendung von Lab für den Reifungsprozeß von Käse oder anderen Milchprodukten. Solche Prozesse können um ein erfindungsgemäßes Protein bereichert oder vollständig von ihm durchgeführt werden. Auch kohlenhydratreiche Lebensmittel oder Lebensmittelrohstoffe für Nicht-Ernährungszwecke, wie beispielsweise Getreidemehl oder Dextrin, können mit entsprechenden Proteasen behandelt werden, um sie von begleitenden Proteinen zu befreien. Auch für solche Anwendungen ist eine erfindungsgemäße Protease geeignet, insbesondere dann, wenn sie unter alkalischen oder leicht denaturierenden Bedingungen durchgeführt werden sollen.

Entsprechendes gilt für die Futtermittelherstellung. Hier kann es neben einer vollständigen Befreiung von Proteinen auch von Interesse sein, die proteinhaltigen Ausgangsstoffe oder Stoffgemische mit Proteasen lediglich kurze Zeit zu behandeln, um sie für die Haustiere leichter verdaulich zu machen. Solch eine Behandlung kann beispielsweise auch für die Herstellung von Medienbestandteilen, etwa für die Fermentation von Mikroorganismen eingesetzt werden.

In einer weitere Ausführungsform dieses Erfindungsgegenstands werden die oben beschriebenen, erfindungsgemäßen Proteine für kosmetische Zwecke eingesetzt.

Beansprucht werden also Kosmetika mit einem oben beschriebenen, erfindungsgemäßen Protein oder Derivat oder kosmetische Verfahren unter Einbeziehung eines oben beschriebenen, erfindungsgemäßen Proteins oder Derivats oder die Verwendung eines oben beschriebenen, erfindungsgemäßen Proteins oder Derivats zu kosmetischen Zwecken, insbesondere im Rahmen entsprechender Verfahren oder in entsprechenden Mitteln.

Denn Proteasen spielen auch im Zellemeuerungsprozeß der menschlichen Haut (Desquamation) eine entscheidende Rolle (T. Egelrud et al., Acta Derm. Venerol., Band 71 (1991), S.471-474). Dementsprechend werden Proteasen auch als bioaktive Komponenten in Hautpflegemitteln verwendet, um den Abbau der in trockener Haut vermehrten Desmosomenstrukturen zu unterstützen, beilspielsweise gemäß der Anmeldungen WO 95/07688 oder WO 99/18219. Der Einsatz von Subtilisin-Proteasen, für kosmetische Zwecke wird beispielsweise in WO 97/07770 beschrieben. Auch erfindungsgemäße Proteasen, insbesondere solche, die etwa nach Mutagenese oder durch Zugabe entsprechender, mit ihnen wechsetwirkender Stoffe in ihrer Aktivität kontrolliert sind, eignen sich als aktive Komponenten in Haut- oder Haar-Reinigungs-oder Pflegemitteln. Besonders bevorzugt sind solche Präparationen dieser Enzyme, die wie oben beschrieben, beispielsweise durch Kopplung an makromolekulare Träger (vergleiche US 5230891) stabilisiert und/oder durch Punktmutationen an hochallergenen Positionen derivatisiert sind, so daß sie für den Menschen eine höhere Hautverträglichkeit aufweisen.

Dementsprechend wird auch die Verwendung derartiger proteolytischer Enzyme zu kosmetischen Zwecken in diesen Erfindungsgegenstand einbezogen, insbesondere in entsprechenden Mitteln, wie beispielsweise Shampoos, Seifen oder Waschlotionen, oder in Pflegemitteln, die beispielsweise in Form von Cremes angeboten werden. Auch die Verwendung in einem schälenden Arzneimittel, beziehungsweise zu dessen Herstellung ist in diesen Anspruch eingeschlossen.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

### Beispiel 1

### Isolierung und Identifizierung eines proteolytisch aktiven Bakterienstamms

0,1 g einer Bodenprobe wurden in 1 ml steriler 0,9 %iger NaCl-Lösung suspendiert und auf milchpulverhaltigen Agarplatten (1,5% Agar, 0,5% NaCl, 0,1 % K₂HPO₄, 0,1 % Hefeextrakt, 2 % Pepton (Firma ICN, Eschwede, Art.-Nr. 104808), 1 % Milchpulver (Skim milk; Firma Difco, Heidelberg, Art.-Nr. 232100), pH 10), ausplattiert. Nach 72-stündiger Inkubation bei 30°C zeigten sich Kolonien mit Klärungshöfen in dem milchigen Agar. Aus diesen wurden Einzelkolonien entnommen und in Horikoshi-Medium (0,1 % K₂HPO₄, 0,5 % Hefeextrakt, 1 % Pepton, 0,02 % MgSO₄, 0,3 % Na₂CO₃, pH 9) in ErlenmeyerKolben bei 37°C und unter Schütteln bei 200 rpm kultiviert.

Einer dieser Klone wurde am 1.3.2001 bei der Deutschen Sammlung von Mikroorganismen und Zellkulturen GmbH, Mascheroder Weg 1b, 38124 Braunschweig (DSMZ) hinterlegt. Er trägt dort die Bezeichnung ID 01-194 und die Eingangsnummer DSM 14393. Die maßgeblichen Angaben über die Merkmale dieses biologischen Materials, wie sie von der DSMZ am 19.4.2001 bei der Hinterlegung bestimmt worden sind, werden in folgender Tabelle 1 zusammengestellt.

**Tabelle 1: Mikrobiologische Eigenschaften des Bacillus gibsonii (DSM 14393). (Bestimmt von der DSMZ am 19.4.2001.)**

| **Eigenschaft** | | **Ergebnis** | |
|---|---|---|---|
| Zellform | | Stäbchen | |
| | Breite [µm] | | 0,8-1,2 |
| | Länge [µm] | | 2,0-3,0 |
| Sporen | | positiv, oval | |

| Sporangium geschwollen | | negativ | |
|---|---|---|---|
| Wachstum CASO, pH 7 | | positiv | |
| Wachstum DSM-Med. 31, pH 9,7 | | positiv | |
| Anaerobes Wachstum | | negativ | |
| VP Reaktion | | negativ | |
| | pH in VP Medium | | 6,4 |
| Maximale Temperatur | | | |
| | Wachstum positiv bei °C | | 30 |
| | Wachstum negativ bei °C | | 40 |
| Wachstum in | | | |
| | Medium pH 5,7 | | negativ |
| | NaCl 2% | | positiv |
| | 5% | | positiv |
| | 7% | | positiv |
| | 10% | | positiv |
| | Lysozym-Medium | | negativ |
| Säure aus (ASS) | | | |
| | D-Glucose | | positiv, schwach |
| | L-Arabinose | | negativ |
| | D-Xylose | | negativ |
| | D-Mannit | | positiv, schwach |
| | D-Fructose | | positiv, schwach |
| Gas aus Glucose | | negativ | |
| Hydrolyse von | | | |
| | Stärke | | negativ |
| | Gelatine | | positiv, schwach |
| | Tween 80 | | negativ |
| | Eskulin | | negativ |
| Verwertung von | | | |
| | Citrat (Koser) | | negativ |
| | Propionat | | negativ |
| NO₂ aus NO₃ | | | positiv |
| Phenylalanindesaminase | | | negativ |
| Arginindihydrolase | | | negativ |
| Alkalische Tests: | | | |
| | 2% bis 5% NaCl | | positiv |
| | Tween 40 | | negativ |
| | Tween 60 | | negativ |
| | Tween 80 | | negativ |
| Muster der zellulären Fettsäuren | | typisch für Genus *Bacillus* | |
| Partielle Sequenzierung der 16 S-rDNA | | 99,4 % Ähnlichkeit zu *B*. *gibsonii* | |

### Beispiel 2

### Klonierung und Sequenzierung der maturen Protease

Chromosomale DNA aus *Bacillus gibsonii* (DSM 14393) wurde nach Standardmethoden präpariert, mit dem Restriktionsenzym *Sau* 3A behandelt und die erhaltenen Fragmente in den Vektor pAWA22 kloniert. Dabei handelt es sich um einen von pBC16 abgeleiteten Expressionsvektor für den Einsatz in Bacillus-Spezies (Bernhard et al. (1978), J. Bacteriol., Band 133 (2), S. 897-903). Dieser Vektor wurde in den Protease-negativen Wirtsstamm *Bacillus subtilis* DB 104 (Kawamura und Doi (1984), J. Bacteriol., Band 160 (1), S. 442-444) transformiert.

Die Transformanten wurden zunächst auf DM3-Medium (8 g/l Agar, 0,5 M Bernsteinsäure, 3,5 g/l K₂HPO₄, 1,5 g/l KH₂PO₄, 20 mM MgCl₂, 5 g/l Casiaminoacids, 5 g/l Hefeextrakt, 6 g/l Glucose, 0,1 g/l BSA) regeneriert und dann auf TBY-Skimmilk-Platten (10 g/l Pepton, 10 g/l Milchpulver (siehe oben), 5 g/l Hefe, 5 g/l NaCl, 15 g/l Agar) überimpft. Proteolytisch aktive Klone wurden anhand ihrer Lysehöfe identifiziert. Aus den erhaltenen proteolytisch aktiven Klonen wurde einer ausgewählt (p/TI-1), dessen Plasmid isoliert und das Insert nach Standardmethoden sequenziert.

Das ca. 2 kb große Insert enthielt einen offenen Leserahmen von ca. 1 kb. Dessen Sequenz ist im Sequenzprotokoll unter der Bezeichnung SEQ ID NO. 1 angegeben. Es umfaßt 1152 bp. Die hiervon abgeleitete Aminosäuresequenz umfaßt 383 Aminosäuren, gefolgt von einem Stop-Codon. Sie ist im Sequenzprotokoll unter SEQ ID NO. 2 angegeben. Hiervon sind die ersten 114 Aminosäuren wahrscheinlich nicht im maturen Protein enthalten, so daß sich für das mature Protein voraussichtlich eine Länge von 269 Aminosäuren ergibt.

Diese Sequenzen wurden im August 2001 mit den aus den allgemein zugänglichen Datenbanken Swiss-Prot (Geneva Bioinformatics (GeneBio) S.A., Genf, Schweiz; http://www.genebio.com/sprot.html) und GenBank (National Center for Biotechnology Information NCBI, National Institutes of Health, Bethesda, MD, USA) erhältlichen Proteasesequenzen verglichen. Dabei wurden als nächstähnliche Enzyme die in der folgenden Tabelle 2 zusammengestellten identifiziert.

**Tabelle 2: Homologie der Alkalischen Protease aus Bacillus gibsonii (DSM 14393) zu den nächstähnlichen und weiteren repräsentativen Proteinen.**

| Darin bedeuten: | | | | | |
|---|---|---|---|---|---|
| ID | Die Eintragungsnummern bei den Datenbanken Genbank und Swiss-Prot; | | | | |
| Ident. DNA | Identität auf DNA-Ebene in %; | | | | |
| Ident. Proprä. | Identität auf Aminosäure-Ebene, bezogen auf das Propräprotein, in %; | | | | |
| Ident. mat. Prot. | Identität auf Aminosäure-Ebene, bezogen auf das mature Protein, in %; | | | | |
| n. nicht | in den Datenbanken angegeben. | | | | |

| **Enzym** | **Organismus** | **ID** | **Ident. DNA** | **Ident. Proprä.** | **Ident. mat. Prot.** |
|---|---|---|---|---|---|
| Subtilisin P92 | *Bacillus alkalophilus* | ELYA_BACAO | 62 | 67 | 80 |
| Subtilisin 309 (Savinase^{®}) | *Bacillus lentus* | SUBS_BACLE | n. | n. | 80 |
| *B. lentus-* | *Bacillus lentus* | SUBB_BACLE | n. | n. | 80 |
| Alkalische | DSM 5483 | | | | |
| Protease | | | | | |
| Alkalische Elastase | *Bacillus Ya-B* | EL Y A_BACSP | 66 | 67 | 78 |
| Subtilisin Sendai | *Bacillus Sendai* | Q45522 | 65 | 65 | 78 |
| Subtilisin AprQ | *Bacillus sp.* | Q45523 | 56 | 49 | 61 |
| Subtilisin Novo | *Bacillus* | SUBT_BACAM ^{.} | n. | 45 | 57 |
| BPN' | *amyloliquefaciens* | | | | |
| Subtilisin AprN | *Bacillus subtilis var. natto* | SUBN_BACNA | 55 | 45 | 56 |
| Subtilisin | *Bacillus amylosacchariticus* | SUBT_BACSA | 54 | 45 | 56 |
| Subtilisin J | *Geobacillus stearothermophilus* | SUBT_BACST | 54 | 44 | 56 |
| Subtilisin | *Bacillus pumilus* | SUBT_BACPU | n. | n. | 55 |
| Subtilisin E | *Bacillus subtilis* | SUBT_BACSU | 54 | 45 | 55 |
| Subtilisin Carlsberg | *Bacillus licheniformis* | SUBT_BACLI | 54 | 42 | 54 |

Die Aminosäuresequenzen dieser Proteasen werden auch im Alignment der Figur 1 miteinander verglichen.

### Beispiel 3

### Aufreinigung und Charakterisierung der Alkalischen Protease

Ein 500-ml Erlenmeyerkolben wurde mit 100 ml Horikoshi-Medium (siehe oben) versetzt, mit einer Kolonie des nach Beispiel 2 transformierten Bacillus-Stamms angeimpft und für 72 h bei 37°C kultiviert, bis die stationäre Wachstumsphase erreicht war.

Aus dem Überstand dieser Kultur konnte über folgende Aufreinigungsschritte ein singuläres proteolytisches Enzym erhalten werden: Dialyse des Überstands gegen 20 mM HEPES/NaOH-Puffer, pH 7,6; negative Anionenaustauschchromatographie an Q-Sepharose^{®} (Firma Pharmacia-Amersham Biotech, Schweden); Kationenaustauschchromatographie des Durchbruchs an S-Sepharose^{®} (Firma Pharmacia-Amersham) bei Elution mit einem Gradientenpuffer von HEPES/NaOH, 0-1 M NaCl, pH 7,6. Die Protease eluierte bei 0,2 M NaCl und wurde anschließend mit einer Kationenaustauschchromatographie an Resource S^{®} (Firma Pharmacia-Amersham) und HEPES/NaOH (pH 7,6) als Eluens aufkonzentriert.

Auf diese Weise wurde ein gemäß SDS-Gelektrophorese und Coomassie-Färbung reines Protein gewonnen.

### Beispiel 4

### SDS-Polyacrylgelelektrophorese und isoelektrische Fokussierung

Bei denaturierender SDS-Polyacrylgelelektrophorese im PHAST^{®}-System der Firma Pharmacia-Amersham Biotech, Schweden, weist die nach Beispiel 2 und 3 erhaltene Alkalische Protease aus *B. gibsonii* (DSM 14393) ein Molekulargewicht von 26 kD auf.

Gemäß isoelektrischer Fokussierung, ebenfalls im PHAST^{®}-System der Firma Pharmacia-Amersham Biotech liegt der isoelektrische Punkt der Alkalischen Protease aus *B. gibsonii* (DSM 14393) bei 11.

### Beispiel 5

### Enzymatische Eigenschaften

### Spezifische Aktivität

Die spezifische Aktivität der nach Beispiel 2 und 3 aufgreinigten Alkalischen Protease aus *B. gibsonii* (DSM 14393) wurde mit dem Substrat Suc-Ala-Ala-Pro-Phe-p-Nitroanilid (AAPF; Firma Bachem Biochemica GmbH, Heidelberg) ermittelt. Während 5minütiger Inkubation bei pH 8,6 und 25°C zeigte sie eine Aktivität von 16 U/mg. Dabei entspricht 1 U 1 µmol gespaltenem Substrat pro Minute.

### pH-Abhängigkeit

Das pH-Profil der Alkalischen Protease aus *Bacillus gibsonii* (DSM 14393) wurde über einen pH-Bereich von 6-12 aufgenommen. Dazu wurden Aktivitäten bei jeweils ganzzahligen pH-Werten mit Casein als Substrat bei 50°C gemessen. Das pH-Optimum liegt demnach bei pH 10. Nach 15minütiger Inkubation bei 50°C liegen bei pH 11 noch 73%, bei pH 12 noch 1%, bei pH 6 noch 16% und bei pH 9 noch 83% Aktivität vor.

### Beispiel 6

### Beitrag zur Waschleistung bei niedriger eingesetzter Aktivität

Für dieses Beispiel wurden standardisiert verschmutze Textilien eingesetzt, die von der Eidgenössischen Material-Prüfungs- und -Versuchsanstalt, St. Gallen, Schweiz (EMPA), oder der Wäschereiforschungsanstalt, Krefeld, bezogen worden waren. Dabei wurden folgende Anschmutzungen und Textilien verwendet: A (Blut/Milch/Tusche auf Baumwolle), B (Blut auf Baumwolle) und C (Ei/Ruß auf Baumwolle).

Mit diesem Testmaterial wurden verschiedene Waschmittelrezepturen launderometrisch auf ihre Waschleistung hin untersucht. Dafür wurde jeweils ein Flottenverhältnis von 1:12 eingestellt und für 30 min bei einer Temperatur von 40°C gewaschen. Die Dosierung lag bei 5,88 g des jeweiligen Mittels pro I Waschflotte. Die Wasserhärte betrug 16° deutscher Härte. In einer parallelen Meßreihe (D) wurden wie C behandelte Proben (Ei/Ruß auf Baumwolle) bei einer Temperatur von 60°C unter ansonsten identischen Bedingungen gewaschen.

Als Kontroll-Waschmittel diente eine Waschmittel-Basis-Rezeptur folgender Zusammensetzung (alle Angaben in Gewichts-Prozent): 4 % lineares Alkylbenzolsulfonat (Natrium-Salz), 4 % C₁₂-C₁₈-Fettalkoholsulfat (Natrium-Salz), 5,5 % C₁₂-C₁₈-Fettalkohol mit 7 EO, 1 % Natrium-Seife, 11 % Natriumcarbonat, 2,5 % amorphes Natriumdisilikat, 20 % Natriumperborat-Tetrahydrat, 5,5 % TAED, 25 % Zeolith A, 4,5 % Polycarboxylat, 0,5 % Phosphonat, 2,5 % Schauminhibitorgranulat, 5 % Natriumsulfat, Rest: Wasser, optischer Aufheller, Salze. Sie wurde für die verschiedenen Versuchsreihen so mit folgenden Proteasen versetzt, daß sich jeweils eine Endkonzentration von 2.250 PE an proteolytischer Aktivität pro I Waschflotte ergab: *B. Ientus*-Alkalische Protease F49 (WO 95/23221; Hersteller: Firma Biozym, Kundl, Österreich), Savinase^{®} (Firma Novozymes A/S, Bagsvaerd, Dänemark), beziehungsweise die erfindungsgemäße Protease aus *B*. *gibsonii* (DSM 14393).

Nach dem Waschen wurde der Weißheitsgrad der gewaschenen Textilien im Vergleich zu dem von Bariumsulfat gemessen, der auf 100 % normiert worden war. Die Messung erfolgte an einem Spektrometer Datacolor SF500-2 bei 460 nm (UV-Sperrfilter 3), 30 mm Blende, ohne Glanz, Lichtart D65, 10°, d/8°. Die erhaltenen Ergebnisse werden als Prozent Remission, das heißt als Prozentangaben im Vergleich zu Bariumsulfat zusammen mit den jeweiligen Anfangswerten in folgender Tabelle 3 zusammengestellt. Angegeben sind die Mittelwerte aus jeweils 4 Messungen. Sie erlauben einen unmittelbaren Rückschluß auf den Beitrag des enthaltenen Enzyms zur Waschleistung des verwendeten Mittels.

**Tabelle 3:**

| **Basiswaschmittel mit** | **A** | **B** | **C** | **D** |
|---|---|---|---|---|
| Anfangswert | 14,3 | 19,9 | 28,9 | 28,9 |
| **Kontrolle ohne Protease** | 22,2 | 67,9 | 50,3 | 55,4 |
| **Erfindungsgemäße Protease aus *B. gibsonii* (DSM 14393)** | 30,4 | 71,0 | 72,3 | 71,5 |
| ***B. Ientus*-Alkalische Protease F49** | 30,2 | 70,8 | 72,8 | 72,3 |
| **Savinase^{®}** | 32,1 | 68,8 | 54,9 | 68,9 |
| Standardabw. | 1,1 | 0,5 | 0,9 | 0,9 |

Man erkennt, daß die erfindungsgemäße Protease aus *B. gibsonii* (DSM 14393) an allen getesteten Anschmutzungen den etablierten Proteasen *B. Ientus*-Alkalische Protease F49 und Savinase^{®} in ihren Beiträgen zur Waschleistung nahekommt und sie zum Teil sogar übertrifft.

### Beispiel 7

### Beitrag zur Waschleistung bei höherer eingesetzter Aktivität

In einer Wiederholung des Versuchs vom vorangegangenen Beispiel wurden die Proteasen unter ansonsten identischen Bedingungen so konzentriert, daß sich pro I Waschflotte jeweils eine Endkonzentration von 5.625 PE an proteolytischer Aktivität ergab.

In folgender Tabelle 4 sind die auf die gleiche Weise erhaltenen Ergebnisse für die gleichen Anschmutzungen A', B' und C', entsprechend A, B und C zusammengestellt.

**Tabelle 4:**

| **Basiswaschmittel mit** | **A'** | **B'** | **C'** |
|---|---|---|---|
| Anfangswert | 14,4 | 20,1 | 29,1 |
| **Kontrolle ohne Protease** | 21,5 | 66,5 | 53,1 |
| **Erfindungsgemäße Protease aus *B. gibsonii* (DSM 14393)** | 37,7 | 73,1 | 74,3 |
| ***B. Ientus*-Alkalische Protease F49** | 32, 8 | 70,0 | 73,4 |
| **Savinase^{®}** | 33,8 | 68,4 | 64,1 |
| Standardabw. | 1,1 | 0,5 | 0,9 |

In diesen Fällen an höherer eingesetzter spezifischer Aktivität zeigt die erfindungsgemäße Protease aus *B. gibsonii* (DSM 14393) bessere oder gleichwertige Leistungen wie die verglichenen etablierten Proteasen.

### Beispiel 8

### Beitrag zur Reinigungsleistung bei niedriger eingesetzter Aktivität

Gefäße mit harten, glatten Oberflächen wurden standardisiert mit Weichei (E), Ei/Milch (F) und hartnäckigem Hackfleisch (G) versehen und mit handelsüblichen Haushaltsgeschirrspülmaschinen gespült. Sie wurden bei 45°C mit dem Normalprogramm der Geschirrspülmaschine Typ Miele^{®} G 676 gespült. Pro Spülgang wurden jeweils 20 g Spülmittel verwendet. Die Wasserhärte betrug 16° deutscher Härte.

Als Spülmittel diente folgende Basis-Rezeptur (alle Angaben jeweils in Gewichts-Prozent): 55 % Natriumtripolyphosphat (berechnet als wasserfrei), 4 % amorphes Natriumdisilikat (berechnet als wasserfrei), 22 % Natriumcarbonat, 9 % Natriumperborat, 2 % TAED, 2 % nichtionisches Tensid, Rest: Wasser, Farbstoffe, Parfüm. Diese Basis-Rezeptur wurde für die verschiedenen Versuche aktivitätsgleich so mit den verschiedenen Proteasen *B. Ientus*-Alkalische Protease F49, Properase^{®}, beziehungsweise der erfindungsgemäßen Protease aus *Bacillus gibsonii* (DSM 14393) versetzt, daß sich jeweils eine Aktivität von 10.000 PE pro Spülgang ergab. Dies entsprach jeweils ca. 0,1 mg Protease-Protein pro g des Reinigungsmittelkonzentrats.

Nach dem Spülen wurde der Abtrag der Anschmutzungen gravimetrisch in Prozent ermittelt. Dafür wurde die Differenz aus dem Gewicht des verschmutzten und anschließend gespülten Gefäßes und dem Anfangsgewicht des Gefäßes in Relation zu der Gewichtsdifferenz des nicht gespülten Gefäßes zum Anfangsgewicht gesetzt. Diese Relation kann als Prozent Abtrag angesehen werden. Für die Anschmutzung G wurde nach dem Spülen eine visuelle Bewertung nach einer Skala von 0 (= unverändert, das heißt sehr starke Anschmutzung) bis 10 (= keinerlei Anschmutzung erkennbar) vorgenommen. Die erhaltenen Ergebnisse werden in folgender Tabelle 5 zusammengestellt. Angegeben sind dort die Mittelwerte aus jeweils 8 Messungen. Sie erlauben einen unmittelbaren Rückschluß auf den Beitrag des enthaltenen Enzyms auf die Reinigungsleistung des verwendeten Mittels.

**Tabelle 5:**

| **Basisspülmittel mit** | E | F | G |
|---|---|---|---|
| **Erfindungsgemäße Protease aus** | 22,3 | 34,7 | 6,7 |
| ***Bacillus gibsonii* (DSM 14393)** | | | |
| ***B. Ientus*-Alkalische Protease F49** | 26,0 | 30,5 | 5,3 |
| **Properase^{®}** | 15,7 | 19,0 | 5,7 |

Diese Ergebnisse zeigen, daß die erfindungsgemäße Protease aus *Bacillus gibsonii* (DSM 14393) hinsichtlich ihrer Leistung in maschinellen Geschirrspülmitteln den anderen getesteten Proteasen teilweise überlegen, zumindest aber ebenbürtig ist; und dies bereits bei einer vergleichsweise niedrigen eingesetzten Aktivität.

### Beispiel 9

### Beitrag zur Reinigungsleistung bei höherer eingesetzter Aktivität

Wie in Beispiel 8 wurden Gefäße standardmäßig mit den Anschmutzungen Weichel (H), Ei/Milch (I) und Milch (J) versehen und auf die gleiche Weise mit den jeweils gleichen Reinigungsmittelrezepturen, bei 45°C mit dem Normalprogramm der Geschirrspülmaschine Typ Miele^{®} G 676 gespült. Der einzige Unterschied bestand darin, daß jeweils 20.000 PE an den jeweiligen Proteasen eingesetzt wurden. Dies entsprach jeweils ca. 0,2 mg Protease im Reinigungsmittelkonzentrat.

Die Ergebnisse für die Proben H und I wurden wie im vorangegangenen Beispiel beschrieben gravimetrisch erhalten. Die Werte für die Anschmutzung J wurden wiederum über visuelle Bewertung nach einer Skala von 0 (= unverändert, das heißt sehr starke Anschmutzung) bis 10 (= keinerlei Anschmutzung erkennbar) vorgenommen. Die erhaltenen Ergebnisse werden in folgender Tabelle 6 zusammengestellt. Angegeben sind dort die Mittelwerte aus jeweils 8 Messungen.

**Tabelle 6:**

| **Basisspülmittel mit** | **H** | **I** | **J** |
|---|---|---|---|
| **Erfindungsgemäße Protease aus *Bacillus gibsonii* (DSM 14393)** | 28,9 | 43,3 | 6,3 |
| ***B. lentus*-Alkalische Protease F49** | 35,1 | 51,5 | 6,1 |
| **Properase®** | 23,1 | 39,4 | 6,1 |

Auch bei höheren eingesetzten Protease-Aktivitäten zeigt sich der höhere oder zumindest vergleichbare Beitrag der erfindungsgemäßen Protease zur Gesamtreinigungsleistung des betreffenden Mittels gegenüber den für maschinelle Geschirrspülmittel etablierten Proteasen *B. Ientus*-Alkalische Protease F49 und Properase^{®}.

### Beschreibung der Figuren

- **Figur 1**:: Alignment der Aminosäuresequenzen der erfindungsgemäßen Protease aus *Bacillus gibsonii* (DSM 14393) mit den nächstähnlichen und den wichtigsten, in Tabelle 2 zusammengestellten bekannten Subtilisinen, jeweils in der maturen, das heißt prozessierten Form.

Folgende Nummern stehen für folgende Proteasen (in Klammern jeweils die ID des Datenbankeintrags; vergleiche auch Tabelle 2 in Beispiel 2):

| | | | |
|---|---|---|---|
| 1 | Erfindungsgemäße Protease | aus *Bacillus gibsonii* (DSM 14393) | |
| 2 | Subtilisin P92 | aus *B. alkalophilus* | |
| | (ELYA_BACAO) | | |
| 3 | Savinase^{®} | aus *B. lentus* | (SUBS_BACLE) |
| 4 | Subtilisin BL | aus *B. lentus* | (SUBB_BACLE) |
| 5 | Alkaline Elastase | aus *B. Ya-B* | (ELYA_BACSP) |
| 6 | Subtilisin Sendai AprS | aus *B. sp.* | (Q45522) |
| 7 | Subtilisin AprQ | aus *B. sp.* | (Q45523) |
| 8 | Subtilisin Novo BPN' | aus *B. amyloliquefaciens* | |
| | (SUBT_BACAM) | | |
| 9 | AprN | aus *B. subtilis var. natto* | |
| | (SUBN_BACNA) | | |
| 10 | Subtilisin | aus *B. amylosacchariticus* | |
| | (SUBT_BACSA) | | |
| 11 | Subtilisin J | aus *Geobacillus stearothermophilus* (SUBT_BACST) | |
| 12 | Subtilisin | aus *B. pumilus* | |
| | (SUBT_BACPU) | | |
| 13 | Subtilisin E | aus *B. subtilis* | |
| | (SUBT_BACSU) | | |
| 14 | Subtilisin Carlsberg | aus *B. licheniformis* | (SUBT_BACLI) |

- **Figur 2**:: Der von pBC16 abgeleitete Expressionsvektor pAWA22, der einen Promotor aus *B. licheniformis* (*PromPLi*) und stromabwärts davon eine *Bcl* I-Restriktionsschnittstelle aufweist (vergleiche Beispiel 2 und Bernhard et al. (1978), J. Bacteriol., 133 (2), S. 897-903).

## Patentansprüche

1. Alkalische Protease vom Subtilisin-Typ, deren Aminosäuresequenz mit der in SEQ ID NO. 2 angegebenen Aminosäuresequenz identisch ist und/oder deren Aminosäuresequenz mit einer von der in SEQ ID NO. 1 angebenen Nukleotidsequenz abgeleiteten Aminosäuresequenz identisch ist.

2. Alkalische Protease vom Subtilisin-Typ, deren Aminosäuresequenz mit der in SEQ ID NO. 2 angegebenen Aminosäuresequenz in den Positionen 115 bis 384 identisch ist und/oder deren Aminosäuresequenz mit einer von der in SEQ ID NO. 1 angebenen Nukleotidsequenz abgeleiteten Aminosäuresequenz in den Positionen 343 bis 1152 identisch ist.

3. Von einer alkalischen Protease vom Subtilisin-Typ nach Anspruch 2 durch Fragmentierung abgeleitetes Protein mit mindestens 260 bereits im Ausgangsmolekül zusammenhängenden, intern der Ausgangsaminosäuresequenz gelegenen Aminosäuren, das an sich Protein zu hydrolysieren vermag.

4. Protein gemäß einem der Ansprüche 1 bis 3, **dadurch gekennzeichnet, daß** es zusätzlich durch Kopplung niedrigmolekularer Verbindungen, durch chemische Umwandlung einer Seitenkette, durch kovalente Bindung von bifunktionellen chemischen Verbindungen und/oder Makromolekülen und/oder durch Vergesellschaftung mit Begleitstoffen derivatisiert ist.

5. Protein gemäß einem der Ansprüche 1 bis 4, **dadurch gekennzeichnet, daß** es zusätzlich stabilisiert ist durch Kopplung an Polymere.

6. Protein nach einem der Ansprüche 1 bis 5, **dadurch gekennzeichnet, daß** es aus einer natürlichen Quelle, insbesondere aus einem Mikroorganismus erhältlich ist.

7. Protein nach Anspruch 6, **dadurch gekennzeichnet, daß** es sich bei dem Mikroorganismus um ein gram-positives Bakterium handelt.

8. Protein nach Anspruch 7, **dadurch gekennzeichnet, daß** es sich bei dem gram-positiven Bakterium um eines der Gattung Bacillus handelt.

9. Protein nach Anspruch 8, **dadurch gekennzeichnet, daß** es sich bei der Bacillus-Spezies um *Bacillus gibsonii,* insbesondere um *Bacillus gibsonii* (DSM 14393) handelt.

10. Nukleinsäure, die für eines der in den Ansprüchen 1 bis 9 bezeichneten Proteine codiert.

11. Nukleinsäure nach Anspruch 10, **dadurch gekennzeichnet, daß** sie aus einer natürlichen Quelle, insbesondere aus einem Mikroorganismus erhältlich ist.

12. Nukleinsäure nach Anspruch 11, **dadurch gekennzeichnet, daß** es sich bei dem Mikroorganismus um ein gram-positives Bakterium handelt.

13. Nukleinsäure nach Anspruch 12, **dadurch gekennzeichnet, daß** es sich bei dem gram-positiven Bakterium um eines der Gattung Bacillus handelt.

14. Nukleinsäure nach Anspruch 13, **dadurch gekennzeichnet, daß** es sich bei der Bacillus-Spezies um *Bacillus gibsonii,* insbesondere um *Bacillus gibsonii* (DSM 14393) handelt.

15. Vektor, der einen in den Ansprüchen 10 bis 14 bezeichneten Nukleinsäurebereich enthält, insbesondere einen, der für eines der in den Ansprüchen 1 bis 9 bezeichneten Proteine codiert.

16. Klonierungsvektor nach Anspruch 15.

17. Expressionsvektor nach Anspruch 15.

18. Wirtszelle die einen in den Ansprüchen 10 bis 14 bezeichneten Nukleinsäurebereich enthält, insbesondere einen, der für eines der in den Ansprüchen 1 bis 9 bezeichneten Proteine codiert, vorzugsweise auf einem Vektor nach einem der Ansprüche 15 bis 17.

19. Wirtszelle, die eines der in Ansprüchen 1 bis 9 bezeichneten Proteine exprimiert oder zu dessen Expression angeregt werden kann, insbesondere unter Einsatz eines in einem der Ansprüche 10 bis 14 bezeichneten Nukleinsäurebereichs, ganz besonders unter Einsatz eines Expressionsvektors gemäß Anspruch 17.

20. Wirtszelle gemäß Anspruch 19, **dadurch gekennzeichnet, daß** sie ein Bakterium ist, insbesondere eines, das das gebildete Protein ins umgebende Medium sekretiert.

21. Bakterium gemäß Anspruch 20, **dadurch gekennzeichnet, daß** es ein gram-positives Bakterium ist, insbesondere eines der Gattung Bacillus, ganz besonders der Species *Bacillus lentus, Bacillus licheniformis, Bacillus amyloliquefaciens, Bacillus subtilis* oder *Bacillus alcalophilus.*

22. Wirtszelle gemäß Anspruch 18 oder 19, **dadurch gekennzeichnet, daß** sie eine eukaryontische Zelle ist, insbesondere eine, die das gebildete Protein posttranslational modifiziert.

23. Verfahren zur Herstellung eines der in den Ansprüchen 1 bis 9 bezeichneten Proteine unter Einsatz einer Nukleinsäure gemäß einem der Ansprüche 10 bis 14 und/oder unter Einsatz eines Vektors gemäß einem der Ansprüche 15 bis 17 und/oder unter Einsatz einer Zelle gemäß einem der Ansprüche 18 bis 22.

24. Mittel, **dadurch gekennzeichnet, daß** es ein Protein nach einem der Ansprüche 1 bis 9 enthält.

25. Wasch- oder Reinigungsmittel, **dadurch gekennzeichnet, daß** es ein Protein nach einem der Ansprüche 1 bis 9 enthält.

26. Mittel nach Anspruch 25, **dadurch gekennzeichnet, daß** es das Protein in einer Menge von 2 µg bis 480 mg, vorzugsweise von 5 µg bis 420 mg, besonders bevorzugt von 20 µg bis 360 mg, ganz besonders bevorzugt von 50 µg bis 240 mg pro g des Mittels enthält.

27. Mittel nach Anspruch 25 oder 26, **dadurch gekennzeichnet, daß** es zusätzlich weitere Enzyme, insbesondere andere Proteasen, Amylasen, Cellulasen, Hemicellulasen, Oxidoreduktasen und/oder Lipasen enthält.

28. Mittel zur Behandlung von Textilrohstoffen oder zur Textilpflege, **dadurch gekennzeichnet, daß** es allein oder neben anderen aktiven Inhaltsstoffen ein Protein nach einem der Ansprüche 1 bis 9 enthält, insbesondere für Fasern oder Textilien mit natürlichen Bestandteilen und ganz besonders für solche mit Wolle oder Seide.

29. Verfahren zur maschinellen Reinigung von Textilien oder von harten Oberflächen, **dadurch gekennzeichnet, daß** in wenigstens einem der Verfahrensschritte ein Protein nach einem der Ansprüche 1 bis 9 aktiv wird, insbesondere in einer Menge von 40 µg bis 96 g, vorzugsweise von 50 µg bis 72 g, besonders bevorzugt von 100 µg bis 48 g und ganz besonders bevorzugt von 200 µg bis 24 g pro Anwendung.

30. Verfahren zur Behandlung von Textilrohstoffen oder zur Textilpflege, **dadurch gekennzeichnet, daß** in wenigstens einem der Verfahrensschritte ein Protein nach einem der Ansprüche 1 bis 9 aktiv wird, insbesondere für Textilrohstoffe, Fasern oder Textilien mit natürlichen Bestandteilen und ganz besonders für solche mit Wolle oder Seide.

31. Verwendung eines proteolytisch aktiven Proteins nach einem der Ansprüche 1 bis 9 zur Reinigung von Textilien oder von harten Oberflächen, insbesondere in einer Menge von 40 µg bis 96 g, vorzugsweise von 50 µg bis 72 g, besonders bevorzugt von 100 µg bis 48 g und ganz besonders bevorzugt von 200 µg bis 24 g pro Anwendung.

32. Verwendung eines Proteins nach einem der Ansprüche 1 bis 9 zur Aktivierung oder Deaktivierung von Inhaltsstoffen von Wasch- oder Reinigungsmitteln.

33. Verwendung eines Proteins nach einem der Ansprüche 1 bis 9 zur biochemischen Analyse oder zur Synthese von niedermolekularen Verbindungen oder von Proteinen.

34. Verwendung eines Proteins nach einem der Ansprüche 1 bis 9 zur Präparation, Reinigung oder Synthese von Naturstoffen oder biologischen Wertstoffen.

35. Verwendung eines Proteins nach einem der Ansprüche 1 bis 9 zur Behandlung von natürlichen Rohstoffen, insbesondere zur Oberflächenbehandlung, ganz besonders in einem Verfahren zur Behandlung von Leder.

36. Verwendung eines Proteins nach einem der Ansprüche 1 bis 9 zur Gewinnung oder Behandlung von Rohmaterialien oder Zwischenprodukten in der Textilherstellung, insbesondere zum Entfernen von Schutzschichten auf Geweben.

37. Verwendung eines Proteins nach einem der Ansprüche 1 bis 9 zur Behandlung von Textilrohstoffen oder zur Textilpflege, insbesondere zur Behandlung von Wolle oder Seide oder woll- oder seidenhaltigen Mischtextilien.

38. Verwendung eines Proteins nach einem der Ansprüche 1 bis 9 zur Behandlung von photographischen Filmen, insbesondere zur Entfernung von gelatinhaltigen oder ähnlichen Schutzschichten.

39. Verwendung eines Proteins nach einem der Ansprüche 1 bis 9 zur Herstellung von Lebensmitteln oder von Futtermitteln.

40. Kosmetikum mit einem Protein nach einem der Ansprüche 1 bis 9 oder ein kosmetisches Verfahren unter Einbeziehung eines Proteins nach einem der Ansprüche 1 bis 9 oder die Verwendung eines Proteins nach einem der Ansprüche 1 bis 9 zu kosmetischen Zwecken, insbesondere im Rahmen entsprechender Verfahren oder in entsprechenden Mitteln.

## Claims

1. An alkaline protease of the subtilisin type, the amino acid sequence of which is identical to the amino acid sequence stated in SEQ ID no. 2 and/or the amino acid sequence of which is identical to an amino acid sequence derived from the nucleotide sequence stated in SEQ ID no. 1.

2. An alkaline protease of the subtilisin type, the amino acid sequence of which is identical in positions 115 to 384 to the amino acid sequence stated in SEQ ID no. 2 and/or the amino acid sequence of which is identical to an amino acid sequence derived from the nucleotide sequence stated in SEQ ID no. 1 in positions 343 to 1152.

3. A protein derived by fragmentation from an alkaline protease of the subtilisin type according to claim 2 having at least 260 amino acids which are already contiguous in the starting molecule and located internally within the initial amino acid sequence, which protein is capable per se of hydrolysing protein.

4. A protein according to any one of claims 1 to 3, **characterised in that** it is additionally derivatised by coupling low molecular weight compounds, by chemical transformation of a side chain, by covalent bonding of difunctional chemical compounds and/or macromolecules and/or by association with accompanying substances.

5. A protein according to any one of claims 1 to 4, **characterised in that** it is additionally stabilised by coupling to polymers.

6. A protein according to any one of claims 1 to 5, **characterised in that** it is obtainable from a natural source, in particular from a microorganism.

7. A protein according to claim 6, **characterised in that** the microorganism is a Gram-positive bacterium.

8. A protein according to claim 7, **characterised in that** the Gram-positive bacterium is a bacterium from the Bacillus genus.

9. A protein according to claim 8, **characterised in that** the Bacillus species is *Bacillus gibsonii,* in particular *Bacillus gibsonii* (DSM 14393).

10. A nucleic acid which encodes one of the proteins designated in claims 1 to 9.

11. A nucleic acid according to claim 10, **characterised in that** it is obtainable from a natural source, in particular from a microorganism.

12. A nucleic acid according to claim 11, **characterised in that** the microorganism is a Gram-positive bacterium.

13. A nucleic acid according to claim 12, **characterised in that** the Gram-positive bacterium is a bacterium from the Bacillus genus.

14. A nucleic acid according to claim 13, **characterised in that** the Bacillus species is *Bacillus gibsonii,* in particular *Bacillus gibsonii* (DSM 14393).

15. A vector which contains a nucleic acid domain designated in one of claims 10 to 14, in particular a domain which encodes one of the proteins designated in claims 1 to 9.

16. A cloning vector according to claim 15.

17. An expression vector according to claim 15.

18. A host cell which contains a nucleic acid domain designated in one of claims 10 to 14, in particular a domain which encodes one of the proteins designated in claims 1 to 9, preferably on a vector according to any one of claims 15 to 17.

19. A host cell which expresses one of the proteins designated in claims 1 to 9 or which may be stimulated to express them, in particular using one of the nucleic acid domains designated in claims 10 to 14, very particularly using an expression vector according to claim 17.

20. A host cell according to claim 19, **characterised in that** it is a bacterium, in particular a bacterium which secretes the protein formed into the surrounding medium.

21. A bacterium according to claim 20, **characterised in that** it is a Gram-positive bacterium, in particular from the Bacillus genus, very particularly from the species *Bacillus lentus, Bacillus licheniformis, Bacillus amyloliquefaciens, Bacillus subtilis* or *Bacillus alcalophilus.*

22. A host cell according to claim 18 or claim 19, **characterised in that** it is a eukaryotic cell, in particular a eukaryotic cell which posttranslationally modifies the protein formed.

23. A method for producing one of the proteins designated in claims 1 to 9 using a nucleic acid according to any one of claims 10 to 14 and/or using a vector according to any one of claims 15 to 17 and/or using a cell according to any one of claims 18 to 22.

24. An agent, **characterised in that** it contains a protein according to any one of claims 1 to 9.

25. A washing or cleaning agent, **characterised in that** it contains a protein according to any one of claims 1 to 9.

26. An agent according to claim 25, **characterised in that** it contains the protein in a quantity of 2 µg to 480 mg, preferably of 5 µg to 420 mg, particularly preferably of 20 µg to 360 mg, very particularly preferably of 50 µg to 240 mg per g of the agent.

27. An agent according to claim 25 or claim 26, **characterised in that** it additionally contains further enzymes, in particular other proteases, amylases, cellulases, hemicellulases, oxidoreductases and/or lipases.

28. An agent for treating textile raw materials or for textile care, **characterised in that** it contains, alone or together with other active ingredients, a protein according to any one of claims 1 to 9, in particular for fibres or textiles comprising natural components and very particularly for those such comprising wool or silk.

29. A method for the machine cleaning of textiles or hard surfaces, **characterised in that** in at least one of the method steps a protein according to any one of claims 1 to 9 becomes active, in particular in a quantity of 40 µg to 96 g, preferably of 50 µg to 72 g, particularly preferably of 100 µg to 48 g and very particularly preferably of 200 µg to 24 g per use.

30. A method for treating textile raw materials or for textile care, **characterised in that** in at least one of the method steps a protein according to any one of claims 1 to 9 becomes active, in particular for textile raw materials, fibres or textiles comprising natural components and very particularly for those comprising wool or silk.

31. Use of a proteolytically active protein according to any one of claims 1 to 9 for cleaning textiles or hard surfaces, in particular in a quantity of 40 µg to 96 g, preferably of 50 µg to 72 g, particularly preferably of 100 µg to 48 g and very particularly preferably of 200 µg to 24 g per use.

32. Use of a protein according to any one of claims 1 to 9 for activating or deactivating ingredients of washing or cleaning agents.

33. Use of a protein according to any one of claims 1 to 9 for biochemical analysis or for synthesising low molecular weight compounds or proteins.

34. Use of a protein according to any one of claims 1 to 9 for preparing, purifying or synthesising natural substances or valuable biological substances.

35. Use of a protein according to any one of claims 1 to 9 for treating natural raw materials, in particular for surface treatment, very particularly in a method for treating leather.

36. Use of a protein according to any one of claims 1 to 9 for obtaining or treating raw materials or intermediates in textiles manufacturing, in particular for removing protective layers on woven fabrics.

37. Use of a protein according to any one of claims 1 to 9 for treating textile raw materials or for textile care, in particular for treating wool or silk or blended textiles.

38. Use of a protein according to any one of claims 1 to 9 for treating photographic films, in particular for removing gelatine-containing protective layers or similar such protective layers.

39. Use of a protein according to any one of claims 1 to 9 for producing foodstuffs or feedstuffs.

40. A cosmetic comprising a protein according to any one of claims 1 to 9 or a cosmetic method involving a protein according to any one of claims 1 to 9 or the use of a protein according to any one of claims 1 to 9 for cosmetic purposes, in particular in the context of corresponding methods or in corresponding agents.

## Revendications

1. Protéase alcaline du type de la subtilisine, dont la séquence d'acides aminés est identique à la séquence d'acides aminés indiquée dans la SEQ ID NO: 2 et/ou dont la séquence d'acides aminés est identique à une séquence d'acides aminés qui dérive de la séquence nucléotidique indiquée dans la SEQ ID NO: 1.

2. Protéase alcaline du type de la subtilisine, dont la séquence d'acides aminés est identique à la séquence d'acides aminés indiquée dans la SEQ ID NO: 2 aux positions 115 à 384 et/ou dont la séquence d'acides aminés est identique à une séquence d'acides aminés qui dérive de la séquence nucléotidique indiquée dans la SEQ ID NO: 1 aux positions 343 à 1152.

3. Protéine dérivée par fragmentation d'une protéase alcaline du type de la subtilisine selon la revendication 2, comprenant au moins 260 acides aminés disposés à l'intérieur de la séquence d'acides aminés de départ, déjà liés dans la molécule de départ, qui possède en soi une capacité d'hydrolyse protéique.

4. Protéine selon l'une quelconque des revendications 1 à 3, **caractérisée en ce qu'**on la dérive en outre par couplage de composés à bas poids moléculaire, par transformation chimique d'une chaîne latérale, par liaison covalente de macromolécules et/ou de composés chimiques bifonctionnels et/ou par association d'adjuvants.

5. Protéine selon l'une quelconque des revendications 1 à 4, **caractérisée en ce qu'**elle est en outre stabilisée par couplage à des polymères.

6. Protéine selon l'une quelconque des revendications 1 à 5, **caractérisée en ce qu'**on l'obtient à partir d'une source naturelle, en particulier à partir d'un micro-organisme.

7. Protéine selon la revendication 6, **caractérisée en ce qu'**il s'agit, en ce qui concerne le micro-organisme, d'une bactérie Gram-positive.

8. Protéine selon la revendication 7, **caractérisée en ce qu'**il s'agit, en ce qui concerne la bactérie Gram-positive, d'une bactérie du genre Bacillus.

9. Protéine selon la revendication 8, **caractérisée en ce qu'**il s'agit, en ce qui concerne le genre Bacillus, de *Bacillus gibsonii,* en particulier de *Bacillus gibsonii* (DSM 14393).

10. Acide nucléique qui code pour une des protéines décrites aux revendications 1 à 9.

11. Acide nucléique selon la revendication 10, **caractérisé en ce qu'**on l'obtient à partir d'une source naturelle, en particulier à partir d'un micro-organisme.

12. Acide nucléique selon la revendication 11, **caractérisé en ce qu'**il s'agit, en ce qui concerne le micro-organisme, d'une bactérie Gram-positive.

13. Acide nucléique selon la revendication 12, **caractérisé en ce qu'**il s'agit, en ce qui concerne la bactérie Gram-positive, d'une bactérie du genre Bacillus.

14. Acide nucléique selon la revendication 13, **caractérisé en ce qu'**il s'agit, en ce qui concerne le genre Bacillus, de *Bacillus gibsonii,* en particulier de *Bacillus gibsonii* (DSM 14393).

15. Vecteur qui contient un domaine d'acide nucléique décrit aux revendications 10 à 14, en particulier un domaine qui code pour une des protéines décrites aux revendications 1 à 9.

16. Vecteur de clonage selon la revendication 15.

17. Vecteur d'expression selon la revendication 15.

18. Cellule hôte qui contient un domaine d'acide nucléique décrit aux revendications 10 à 14, en particulier un domaine qui code pour une des protéines décrites aux revendications 1 à 9, de préférence sur un vecteur selon l'une quelconque des revendications 15 à 17.

19. Cellule hôte qui exprime une des protéines décrites aux revendications 1 à 9 ou qui peut être activée pour son expression, en particulier par la mise en oeuvre d'un domaine d'acide nucléique décrit à l'une quelconque des revendications 10 à 14, de manière tout à fait particulière par la mise en oeuvre d'un vecteur d'expression selon la revendication 17.

20. Cellule hôte selon la revendication 19, **caractérisée en ce qu'**elle représente une bactérie, en particulier une bactérie qui sécrète la protéine formée dans le milieu environnant.

21. Bactérie selon la revendication 20, **caractérisée en ce qu'**il s'agit d'une bactérie Gram-positive, en particulier d'une bactérie du genre Bacillus, de manière tout à fait particulière de l'espèce *Bacillus lentus, Bacillus licheniformis, Bacillus amyloliquefaciens, Bacillus subtilis* ou *Bacillus alcalophilus.*

22. Cellule hôte selon la revendication 18 ou 19, **caractérisée en ce qu'**elle représente une cellule eucaryote, en particulier une cellule eucaryote qui soumet la protéine obtenue à une modification posttraductionnelle.

23. Procédé pour la préparation d'une protéine décrite aux revendications 1 à 9 par la mise en oeuvre d'un acide nucléique selon l'une quelconque des revendications 10 à 14 et/ou par la mise en oeuvre d'un vecteur selon l'une quelconque des revendications 15 à 17 et/ou par la mise en oeuvre d'une cellule selon l'une quelconque des revendications 18 à 22.

24. Agent **caractérisé en ce qu'**il contient une protéine selon l'une quelconque des revendications 1 à 9.

25. Agent de lavage ou de nettoyage, **caractérisé en ce qu'**il contient une protéine selon l'une quelconque des revendications 1 à 9.

26. Agent selon la revendication 25, **caractérisé en ce qu'**il contient la protéine en une quantité de 2 µg à 480 mg, de préférence de 5 µg à 420 mg, de manière particulièrement préférée de 20 µg à 360 mg, de manière tout particulièrement préférée de 50 µg à 240 mg par gramme de l'agent.

27. Agent selon la revendication 25 ou 26, **caractérisé en ce qu'**il contient en outre des enzymes supplémentaires, en particulier d'autres protéases, amylases, cellulases, hémicellulases, oxydoréductases et/ou lipases.

28. Agent pour le traitement de matières premières textiles ou pour l'entretien des textiles, **caractérisé en ce qu'**il contient, à titre individuel ou à côté d'autres substances actives, une protéine selon l'une quelconque des revendications 1 à 9, en particulier pour des fibres ou des textiles comprenant des constituants naturels et de manière tout à fait particulière pour des fibres ou des textiles comprenant de la laine ou de la soie.

29. Procédé pour le nettoyage mécanique de textiles ou de surfaces dures, **caractérisé en ce qu'**on active, dans au moins une des étapes opératoires, une protéine selon l'une quelconque des revendications 1 à 9, en particulier en une quantité de 40 µg à 96 g, de préférence de 50 µg à 72 g, de manière particulièrement préférée de 100 µg à 48 g, et de manière tout particulièrement préférée de 200 µg à 24 g par utilisation.

30. Procédé pour le traitement de matières premières textiles ou pour l'entretien des textiles, **caractérisé en ce qu'**on active, dans au moins une des étapes opératoires, une protéine selon l'une quelconque des revendications 1 à 9, en particulier pour des matières premières textiles, des fibres ou des textiles comprenant des constituants naturels et de manière tout à fait particulière pour des matières premières textiles, des fibres ou des textiles comprenant de la laine ou de la soie.

31. Utilisation d'une protéine à activité protéolytique selon l'une quelconque des revendications 1 à 9 pour le nettoyage de textiles ou de surfaces dures, en particulier en une quantité de 40 µg à 96 g, de préférence de 50 µg à 72 g, de manière particulièrement préférée de 100 µg à 48 g, et de manière tout particulièrement préférée de 200 µg à 24 g par utilisation.

32. Utilisation d'une protéine selon l'une quelconque des revendications 1 à 9, pour l'activation ou la désactivation de constituants d'agents de lavage ou de nettoyage.

33. Utilisation d'une protéine selon l'une quelconque des revendications 1 à 9, pour l'analyse biochimique ou pour la synthèse de composés à bas poids moléculaire ou de protéines.

34. Utilisation d'une protéine selon l'une quelconque des revendications 1 à 9, pour la préparation, le nettoyage ou la synthèse de substances naturelles ou de matières biologiques de valeur.

35. Utilisation d'une protéine selon l'une quelconque des revendications 1 à 9, pour le traitement de matières premières naturelles, en particulier pour le traitement superficiel, de manière tout à fait particulière, dans un procédé pour le traitement du cuir.

36. Utilisation d'une protéine selon l'une quelconque des revendications 1 à 9, pour l'obtention ou le traitement de matières premières ou de produits intermédiaires dans la fabrication des textiles, en particulier pour l'élimination de couches de protection sur des tissus.

37. Utilisation d'une protéine selon l'une quelconque des revendications 1 à 9, pour le traitement de matières premières textiles ou pour l'entretien des textiles, en particulier pour le traitement de la laine ou de la soie ou de textiles mixtes contenant de la laine ou de la soie.

38. Utilisation d'une protéine selon l'une quelconque des revendications 1 à 9, pour le traitement de films photographiques, en particulier pour éliminer des couches de gélatine ou des couches de protection analogues.

39. Utilisation d'une protéine selon l'une quelconque des revendications 1 à 9, pour la préparation d'aliments ou d'aliments pour animaux.

40. Cosmétique comprenant une protéine selon l'une quelconque des revendications 1 à 9 ou bien procédé cosmétique avec incorporation d'une protéine selon l'une quelconque des revendications 1 à 9 ou bien utilisation d'une protéine selon l'une quelconque des revendications 1 à 9 à des fins cosmétiques, en particulier dans le cadre de procédés correspondants ou dans des agents correspondants.
